# EUROPEAN PATENT SPECIFICATION

(11) **EP 1 399 577 B1**
(45) Date of publication and mention of the grant of the patent: **07.02.2007**
(21) Application number: 02741268.3
(22) Date of filing: 24.06.2002
(51) Int. Cl.: C12P 13/04, C12P 13/22, C12P 17/00

(54) **METHOD FOR PRODUCING L-AMINO ACIDS**
VERFAHREN ZUR HERSTELLUNG VON L-AMINOSÄUREN
PROCEDE DE PRODUCTION DE L-AMINO-ACIDES

(30) Priority: 25.06.2001 JP 2001190867; 30.11.2001 JP 2001367780
(43) Date of publication of application: 24.03.2004
(73) Proprietor: Showa Denko K.K., Tokyo 105-8518 (JP)
(72) Inventor: Aoki, Hirobumi, Showa Denko, Midori-ku, Chiba-shi, Chiba 267-0056 (JP); Kamachi, Harumi, Showa Denko, Midori-ku, Chiba-shi, Chiba 267-0056 (JP)
(74) Representative: Strehl Schübel-Hopf & Partner
(86) International application number: PCT/JP2002/006287
(87) International publication number: WO 2003/000915

(56) References cited:
- US-A- 5 981 239
- LIANG X ET AL: "DEVELOPMENTAL AND ENVIRONMENTAL REGULATION OF A PHENYLALANINE AMMONIA-LYASE-BETA-GLUCURONIDASE GENE FUSION IN TRANSGENIC TOBACCO PLANTS" PROCEEDINGS OF THE NATIONAL ACADEMY OF SCIENCES OF USA, NATIONAL ACADEMY OF SCIENCE. WASHINGTON, US, vol. 86, 1 December 1989 (1989-12-01), pages 9284-9288, XP002052657 ISSN: 0027-8424
- LEE ET AL.: "Truncated Phenylalanine Ammonia-lyase Expression in Tomato (Lycopersicon esculentum)" J. BIOL. CHEM., vol. 267, no. 17, 15 June 1992 (1992-06-15), pages 11824-11830, XP002216889 cited in the application
- PATENT ABSTRACTS OF JAPAN vol. 010, no. 203 (C-360), 16 July 1986 (1986-07-16) & JP 61 043993 A (KYOWA HAKKO KOGYO CO LTD), 3 March 1986 (1986-03-03) cited in the application
- PATENT ABSTRACTS OF JAPAN vol. 012, no. 410 (C-540), 28 October 1988 (1988-10-28) & JP 63 148992 A (ASAHI GLASS CO LTD), 21 June 1988 (1988-06-21) cited in the application

## Description

### CROSS REFERENCES OF RELATED APPLICATION

This application is an application filed uner 35 U. S . C. §111(a) claiming benefit pursuant to 35 U.S.C. §119(e) (1) of the filing date of Provisional Applications 60/303, 086 filed on July 6, 2001 and 60/339,389 filed on December 14, 2001 pursuant to 35 U.S.C. §111(b).

### BACKGROUND OF THE INVENTION

The present invention relates to a method for producing an optically active amino acid, which comprises adding ammonia to an acrylic acid derivative by action of a plant enzyme to thereby obtain the corresponding optically active amino acid. The optically active amino acid is useful as a synthesis material for pharmaceuticals, agricultural chemicals and other fine chemicals.

There have been studied a large number of methods for obtaining optically active amino acids such as organic synthesis reaction involving the use of an asymmetric catalyst and a fermentative production method involving the use of the specificity of a microorganism.

There are many publications such as UK Patent No. 1489468 and Japanese Patent Laid-Open No. 53-96388 (1978) reporting a method for producing an optically active phenylalanine by optical selection, in which phenylalanine ammonia-lyase derived from a microorganism or a microorganism itself having the enzyme activity is allowed to act on cinnamic acid. This method uses a reaction of adding an amino group to an α-carbon of cinnamic acid by the action of the enzyme at a high ammonia concentration, and thereby L-phenylalanine can be generated with high optical purity.

As a method for obtaining an optically active L-phenylalanine derivative having a substituent on a phenyl group thereof, there is considered a method of introducing a substituent onto the phenyl group, applying various types of modification reaction to L-phenylalanine. By this method, however, inevitable is reduction of the yield due to side reaction occurring in parts other than the phenyl group and decrease of optical purity, because of progression of racemization under extremely severe reaction conditions. Moreover, since it is difficult to obtain the position specificity of a substituent to be introduced on a phenyl group, it results in low yield or difficulty of separation and purification. Thus, this method is not practical.

Here is considered another method of allowing the above described phenylalanine ammonia-lyase to act on a cinnamic acid derivative having a substituted phenyl group, to which a desired substituent has previously been introduced. Since an enzyme reaction proceeds under moderate conditions in this method, it provides no detrimental effects caused by a side reaction to a phenyl group and a substituent on the group, and hence it is expected that an optically active L-phenylalanine derivative with high purity is provided by this method.

However, since the substrate specificity of phenylalanine ammonia-lyase derived from a microorganism is generally strict, in most cases this enzyme has a significantly low or almost no reactivity to a substituted phenyl group, in comparison with the original reactivity from cinnamic acid to L-phenylalanine. There are only a few disclosed cases such as a method of obtaining optically active fluorinated phenylalanine by action of the above enzyme, using, as a starting material, a cinnamic acid derivative having a fluorinated phenyl group (Japanese Patent Laid-Open No. 63-148992 (1988) or a method of obtaining a phenylalanine derivative by using a specific microorganism of *Rhodotorula* (US Patent No. 5981239). Applicable compounds are limited and the productivity is not industrially sufficient.

Generally, it is known that phenylalanine ammonia-lyase is widely distributed in the living world as a whole such as animals, plants and microorganisms. That is to say, examples of microorganisms comprising this enzyme include *Rhodotorula rubra* (Japanese Patent Laid-Open No. 61-043993 (1986), GB1489468), *Rhodosporidium toruloides* (Japanese Patent Laid-Open No. 60-227670(1985)), *Cladosporidium cladosporioides* (Japanese Patent Laid-Open No. 62-111687 (1987)), etc., and apart from these examples, many other microorganisms have been reported.

Examples of plants comprising phenylalanine ammonia-lyase include thale-cress (*Arabidopsis thaliana*)*,* tea *(Camellia sinensis),* chick-pea (*Cicer arietinum*)*,* lemon *(Citrus limonis),* cucumber *(Cucumis sativus* L.), carrot (*Daucus carota*), murasaki (*Lithospermum erythrorhizon*), tomato (*Lycopersicon esculentum*), tobacco (*Nicotiana tabacum)*, rice *(Oryza sativa*), parsley *(Petroselinum crispum, Petroselinum hortense*)*,* loblolly pine *(Pinus taeda*)*,* poplar (*Populus kitakamiensis,* etc.), cherry (*Prunus avium*)*,* bramble *(Rubus idaeus*), eggplant *(Solanum tuberosum),* stylo *(Stylosanthes humilis*), hop clover (*Trifolium subterrane*), grape (*Vitis vinifera*), bush bean (*Phaseolus vulgaris*), etc.

There are many reports regarding a structural gene of phenylalanine ammonia-lyase derived from a plant, pal (hereinafter, referred to as "pal gene"). That is, examples of the pal gene determined include those of rice (*Oryza sativa, Biochim. Biophys. Acta* (1993), 1171(3), 321-322, *Plant Mol. Biol.* (1995), 29(3), 535-550), tea *(Camellia sinensis, Theor. Appl. Genet.* (1994), 89(6), 671-675), thale-cress (*Arabidopsis thaliana, Plant Mol. Biol.* (1995), 27, 327-338), murasaki (*Lithospermum erythrorhizon, Biosci. Biotech. Biochem*. (1997), 61(12), 1995-2003), tomato (*Lycopersicon esculentum, J. Biol. Chem.* (1992), 267, 11824-11830), hop clover (*Trifolium subterraneum, Gene* (1994), 138 (1-2), 87-92), pea *(Pisum sativum, Plant Mol. Biol.* (1992), 20(1), 167-170, Japanese Patent Application Laid-Open (Kokai) No. 5-153978 (1993)), poplar *(Populus trichocarpa, Plant Physiol.* (1993), 102, 71-83), tobacco *(Nicotiana tabacum, Plant Mol. Biol.* (1996), 30, 711-722), soybean *(Glycinemax, DNA Sequence* (1991), 1(5), 335-346), sweet potato *(Ipomoea batatas, Plant Physiol.* (1989), 90(4), 1403-1407), wheat *(Triticum aestivum),* parsley *(Petroselinum crispum),* sunflower *(Helianthus annuus),* alfalfa *(Medicago sativa),* etc., and the number of the known pal gene reaches more than 40 kinds including those of the isomers in the same species or the partial sequences.

Phenylalanine ammonia-lyases derived from a plant mutually have many conservative sequences; their amino acid sequences show 50% or more homology at the lowest degree. Hence it can be predicted that there are many commonalties in the functions and properties.

Phenylalanine ammonia-lyase derived from a plant is known as an initial enzyme of a phenylpropanoid and isoflavonoid synthetic pathway in the secondary metabolism of a plant and also an enzyme catalyzing a reaction in a rate determining step, i.e. a deamination reaction of phenylalanine. Since this enzyme and a gene thereof are considered to be associated with stress resistance of plants, the functions of this enzyme have thoroughly been studied. Moreover, both basic and applied attempts to allow for the pal gene expression in a plant body to produce a disease resistance plant have broadly been made.

Although many research reports exist as stated above, differently from the same enzyme derived from a microorganism, regarding studies from the viewpoint of production of a substance, attempts to produce an organic compound that can be used as a common industrial material have not been made, except for an attempt to increase the content of some useful substances of phenylpropanoids or isoflavonoids (*Planta.* (1979), 14(6), 369-376, *Biochim. Biophys. Acta.* (1979), 563, 278-292). Needless to say, there have never been reported attempts to produce a substance by use of a reverse reaction, i.e. amination reaction. In the first place, not only the present enzyme but also other enzymes derived from a plant have seldom been used for production of a substance, because there is a generally accepted notion that the practicability of these enzymes, in view of low stability, high substrate specificity, etc., is lower than that of enzymes derived from a microorganism. Accordingly, when those skilled in the art conceived a method for producing an L-phenylalanine derivative, one of an organic compound that was similar to the present invention, an enzyme derived from a plant was one of the least noticeable materials for them, to the extent that an enzyme having the same catalytic function was present in other sources.

### SUMMARY OF THE INVENTION

It is one of the objects of the present invention to provide a new method for conveniently and efficiently obtaining an L-amino acid with high optical purity. That is, one of the objects of the present invention is to provide a new method for simply and efficiently obtaining an L-amino acid with high optical purity, using, as a starting material, an acrylic acid derivative such as a cinnamic acid derivative having a substituent on a phenyl group.

The present inventors have focused attention on the broad substrate specificity of phenylalanine ammonia-lyase derived from a plant and have studied on the activity of the enzyme to generate various amino acids, which are industrially useful.

First, ammonia-lyase having high reactivity with a cinnamic acid derivative having a substituent on a phenyl group thereof, has been searched throughout the living world as a whole such as animals, plants and microorganisms.

As a result, the present inventors have found that a phenylalanine ammonia-lyases derived from a plant has a much higher activity to generate a phenylalanine derivative than the same enzymes derived from a microorganism, which have previously been known. As stated above, the step of generating phenylpropanoid, isoflavonoids or the like in a plant body, with which these enzymes are associated, has thoroughly been studied, but such studies relate to generation of a cinnamic acid derivative due to an elimination reaction in which phenylalanine leaves an amino group. The reverse reaction, that is, the possibility of generation of a phenylalanine derivative due to addition of an amino group to a cinnamic acid derivative by the enzymes derived from a plant, has not been studied.

Moreover, the present , inventors have focused attention on the broad substrate specificity of phenylalanine ammonia-lyase derived from a plant and have studied on the activity of the enzyme to generate various types of industrially useful amino acids as well as a phenylalanine derivative. As a result, the present inventors have found that, this enzyme, using as a substrate, an acrylic acid derivative which has an aromatic ring structure that may have substituents and may comprise a hetero atom, acts to generate the corresponding L-amino acid.

As a result of further intensive studies, the present inventors have found that an amino acid can be produced with extremely high efficiency by using a microorganism showing high expression of the pal gene derived from a plant, thereby accomplishing the present invention.

The ability of phenylalanine ammonia-lyase derived from a plant to catalyze the reaction that yields an amino acid, using as a substrate, an acrylic acid derivative having a structure represented by the above general formula (1), has not ever been known, and this is a new finding obtained by the present inventors.

Moreover, the ability of phenylalanine ammonia-lyase derived from a plant to catalyze the reaction that produces a phenylalanine derivative, using as a substrate, a cinnamic acid derivative having various substituents on a phenyl group thereof represented by the above general formula (3), has not ever been known, and this is a new finding obtained by the present inventors.

Furthermore, the ability of phenylalanine ammonia-lyase derived from a plant to catalyze the reaction that yields an L-amino acid, using as a substrate, an acrylic acid derivative having a heterocyclic substituent represented by the above general formula (4) or (5), has not ever been known, and this is a new finding obtained by the present inventors.

That is to say, the present invention includes the following aspects [1] to [30]:
[1] A method for producing an L-amino acid, which comprises, in the presence of ammonia, allowing phenylalanine ammonia-lyase derived from a plant to act on an acrylic acid derivative represented by the following general formula (1): 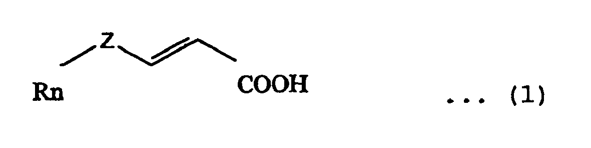 wherein
   Z represents an aromatic ring group that may comprise a hetero atom,
   R represents a substituent on the aromatic ring, and
   n represents an integer of 0 or greater, and where n is 2 or greater, each R may be identical or different;
   the L-amino acid being represented by the following general formula (2): 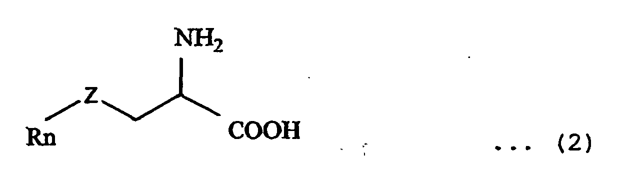 wherein
   Z represents an aromatic ring group that may comprise a hetero atom,
   R represents a substituent on the aromatic ring,
   n represents an integer of 0 or greater, and where n is 2 or greater, each R may be identical or different.
[2] The method for producing an L-amino acid according to [1] above, wherein Rn-Z- is represented by the following general formula (3) : 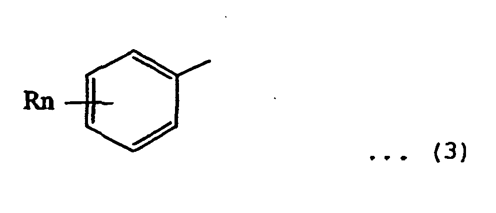 wherein
   R is a substituent on a benzene ring and represents a cyano group, hydroxyl group, carboxyl group, amide group, fluorine atom, chlorine atom, bromine atom, amino group, nitro group, hydroxymethyl group, or alkyl or alkoxy group having 1 to 6 carbon atoms, and
   n represents an integer of 0 to 5, and preferably an integer of 1 to 5. Where n is 2 or greater, each R may be identical or different.
[3] The method for producing an L-amino acid according to [1] above, wherein Rn-Z- is represented by the following general formula (4): 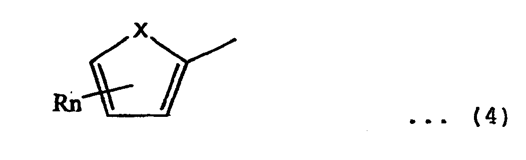 wherein
   X represents S, O, NH or NR¹,
   R¹ represents an alkyl group having 1 to 6 carbon atoms,
   R is a substituent on a hetero ring and represents a cyano group, hydroxyl group, carboxyl group, amide group, fluorine atom, chlorine atom, bromine atom, amino group, nitro group, hydroxymethyl group, or alkyl or alkoxy group having 1 to 6 carbon atoms, and
   n represents an integer of 0 to 3, and where n is 2 or greater, each R may be identical or different.
[4] The method for producing an L-amino acid according to [1] above, wherein Rn-Z- is represented by the following general formula (5): 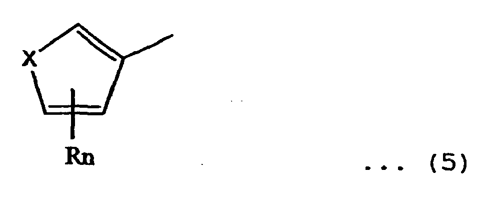 wherein
   X represents S, O, NH or NR¹,
   R¹ represents an alkyl group having 1 to 6 carbon atoms,
   R is a substituent on a hetero ring and represents a cyano group, hydroxyl group, carboxyl group, amide group, fluorine atom, chlorine atom, bromine atom, amino group, nitro group, hydroxymethyl group, or alkyl or alkoxy group having 1 to 6 carbon atoms, and
   n represents an integer of 0 to 3, and where n is 2 or greater, each R may be identical or different.
[5] The method for producing an L-amino acid according to any one of [1] to [4] above, which comprises using a composition obtained by treating plant tissues containing phenylalanine ammonia-lyase.
[6] The method for producing an L-amino acid according to any one of [1] to [4] above, which comprises using a plant cultured cell containing a phenylalanine ammonia-lyase and/or a treated composition thereof.
[7] The method for producing an L-amino acid according to any one of [1] to [4] above, which comprises allowing a phenylalanine ammonia-lyase gene derived from a plant to be expressible in a plant and using transformed plant cultures or a treated product of the plant cultures.
[8] The method for producing an L-amino acid according to any one of [1] to [4] above, which comprises allowing a phenylalanine ammonia-lyase gene derived from a plant to be expressible in a microorganism and using a transformed microorganism culture, a treated product thereof, or an enzyme obtained from the culture.
[9] The method for producing an L-amino acid according to [7] or [8] above, wherein the phenylalanine ammonia-lyase gene derived from a plant is a gene encoding an amino acid sequence having 70% or more homology with the amino acid sequence deduced from the nucleotide sequence of pa12 gene derived from *Lithospermum erythrorhizon.*
[10] The method for producing an L-amino acid according to [7] or [8] above, wherein the phenylalanine ammonia-lyase gene derived from a plant is a gene encoding an amino acid sequence having 80% or more homology with the amino acid sequence deduced from the nucleotide sequence of pal2 gene derived from *Lithospermum erythrorhizon.*
[11] The method for producing an L-amino acid according to any one of [1] to [8] above, wherein the plant from which the phenylalanine ammonia-lyase gene is derived is *Lithospermum* and/or *Camellia.*
[12] The method for producing an L-amino acid according to any one of [8] to [11] above, wherein the microorganism is a bacterium, yeast and/or filamentous fungi.
[13] The method for producing an L-amino acid according to [12] above, wherein the bacterium is *Escherichia coli.*
[14] The method for producing an L-amino acid according to any one of [2] and [5] to [13] above, wherein, in the above general formula (3), at least one of substituents R is a hydroxyl group.
[15] The method for producing an L-amino acid according to any one of [2] and [5] to [13] above, wherein, in the above general formula (3), at least one of substituents R is a cyano group.
[16] The method for producing an L-amino acid according to any one of [2] and [5] to [13] above, wherein, in the above general formula (3), at least one of substituents R is a carboxyl group.
[17] The method for producing an L-amino acid according to any one of [2] and [5] to [13] above, wherein, in the above general formula (3), at least one of substituents R is an amide group.
[18] The method for producing an L-amino acid according to any one of [2] and [5] to [13] above, wherein, in the above general formula (3), at least one of substituents R is a halogen group.
[19] The method for producing an L-amino acid according to any one of [2] and [5] to [13] above, wherein, in the above general formula (3), at least one of substituents R is an amino group.
[20] The method for producing an L-amino acid according to any one of [2] and [5] to [13] above, wherein, in the above general formula (3), at least one of substituents R is a nitro group.
[21] The method for producing an L-amino acid according to any one of [2] and [5] to [13] above; wherein, in the above general formula (3), at least one of substituents R is a hydroxymethyl group.
[22] The method for producing an L-amino acid according to any one of [2] and [5] to [13] above, wherein, in the above general formula (3), at least one of substituents R is an alkyl group having 1 to 6 carbon atoms.
[23] The method for producing an L-amino acid according to any one of [1] to [13] above, wherein R is any one of a cyano group, a hydroxyl group, a nitro group and a carboxyl group, and n is 1.
[24] The method for producing an L-amino acid according to anyone of [2] and [5] to [13] above, wherein the L-amino acid is L-phenylalanine.
[25] The method for producing an L-amino acid according to any one of [3] and [5] to [13] above, wherein, in the above general formula (4), X is O.
[26] The method for producing an L-amino acid according to any one of [3] and [5] to [13] above, wherein, in the above general formula (4), X is S.
[27] The method for producing an L-amino acid according to any one of [3] and [5] to [13] above, wherein, in the above general formula (4), X is NH.
[28] The method for producing an L-amino acid according to any one of [4] to [13] above, wherein, in the above general formula (5), X is O.
[29] The method for producing an L-amino acid according to any one of [4] to [13] above, wherein, in the above general formula (5), X is S.
[30] The method for producing an L-amino acid according to any one of [4] to [13] above, wherein, in the above general formula (5), X is NH.

### BRIEF DESCRIPTION OF THE DRAWINGS

FIG. 1 shows the construct of a *Lithospermum erythrorhizon* PAL expression plasmid showing LePAL1 and LePAL2;
FIG. 2 shows the construct of a *Camellia sinensis* PAL expression plasmid showing CAMPAL; and
FIG. 3 shows an example of homology in PAL amino acid sequences.

### DETAILED DESCRIPTION OF THE PREFERRED EMBODIMENTS

The present invention will be described in detail below.

By the method for producing an L-amino acid of the present invention, an optically active amino acid can simply be obtained from an acrylic acid derivative, which may have an aromatic ring group that may have various substituents and may comprise a hetero atom, by a single step of enzyme reaction.

Moreover, by the method for producing an L-amino acid of the present invention, an optically active amino acid can simply be obtained from an acrylic acid derivative, which has a benzene ring group having various substituents, by a single step of enzyme reaction.

A substituent introduced into a benzene ring in advance, e.g. a cyano group, hydroxyl group, carboxyl group, amide group, halogen atom, amino group, nitro group, hydroxymethyl group or alkyl group having 1 to 6 carbon atoms, is retained until completion of a reaction without undergoing any adverse influence under moderate conditions of the reaction, and the corresponding desired L-phenylalanine derivative can be obtained with a good yield at a conversion rate of almost 100%.

Furthermore, by the method for producing an L-amino acid of the present invention, optically active amino acid having a heterocyclic ring structure at a side chain thereof can simply be obtained from an acrylic acid derivative having a heterocyclic ring structure at a side chain thereof, by a single step of enzyme reaction.

The heterocyclic ring structure such as a furyl group, thienyl group, pyrrole group or the like in the above acrylic acid derivative is retained until completion of a reaction without undergoing any adverse influence under moderate conditions of the reaction, and the corresponding desired L-amino acid can be obtained with a good yield at a conversion rate of almost 100%.

An acrylic acid derivative having various substituents used as a reaction material in the present invention can easily be prepared by, what is called, a method by Perkin' s reaction which allows an aldehyde group of an aldehyde derivative into which any given substituent is introduced, to react with acetic anhydride (refer to *Arch . Pharm.* (Weinheim) (1994), 327 (10), 619-625, etc.); a method involving a reaction of malonic acid in the presence of piperidine in a pyridine solvent (refer to *J. Chem. Soc.* (1939), 357-360, etc.); and various other improved methods (refer to *Synth. Commun.* (1999), 29(4), 573-581, etc.)

Plants providing a phenylalanine ammonia-lyase activity, which are applied in the present invention, are not particularly limited. As before mentioned, specific examples include thale-cress *(Arabidopsis thaliana),* tea *(Camellia sinensis*), chick-pea (*Cicer arietinum*), lemon (*Citrus limonis*), cucumber *(Cucumis sativus* L.), carrot (*Daucus carota*), murasaki (*Lithospermum erythrorhizon*), tomato *(Lycopersicon esculentum*), tobacco *(Nicotiana tabacum),* rice (*Oryza sativa*), parsley *(Petroselinum crispum, Petroselinum hortense),* loblolly pine *(Pinus taeda),* poplar *(Populus kitakamiensis,* etc.), cherry *(Prunus avium),* bramble *(Rubus idaeus),* eggplant *(Solanum tuberosum),* stylo (*Stylosanthes humilis),* hop clover *(Trifolium subterrane),* grape (*Vitis vinifera*), bush bean *(Phaseolus vulgaris),* etc., but this enzyme is almost universally present in plants.

In one embodiment of the present invention, phenylalanine ammonia-lyase separated from a plant is used. That is to say, there is provided, as a reaction catalyst, the above enzyme which is separated and purified by known conventional methods of separating and purifying an enzyme from a plant, e.g. application of acetone precipitation, ammonium sulfate precipitation, various separation columns, etc. on an extract from ground plant bodies. As for a specific method of separating and purifying phenylalanine ammonia-lyase from various types of plant bodies, there are known a report by J. Koukol et al. *(J. Biol. Chem.* (1961), 236(10), 2692-2698), and a report by E. A. Havir et al. *(Biochemistry* (1973), 12, 1583-), etc.

In another embodiment of the present invention, a treated product of the above described plant bodies is provided for a reaction of interest. Examples of the treated product of the plant bodies which is used for a reaction include a ground product, a freeze-dried product or an extract from plant bodies; a product obtained by concentrating and extracting an ingredient catalyzing a reaction of interest from the extract; a product obtained by immobili zing the treated product, the extract or the extracted ingredient of the plant bodies on a hardly soluble carrier; etc.

Examples of such an immobilizing carrier include polyacrylic acid, polymethacrylic acid, polyacrylamide, polyvinyl alcohol, poly-N-vinylformamide, polyallylamine, polyethyleneimine, methylcellulose, glucomannan, alginate, carrageenan, and the (crosslinked) copolymers of these compounds. In other words, a compound that forms a water-insoluble solid comprising a plant extract ingredient can be used singly or in a mixed form.

In another embodiment of the present invention, the cultured cell of the above described plant is used. That is, a plant cell is cultured by a common method of obtaining a cultured plant cell. For example, a plant cell is cultured in various knownmedia for plant cell culture such as a Murashige & Skoog complete medium, an LS medium, an M9 medium and a Gamborg B-5 medium that are selected depending on the type of plant, in the presence of a plant hormone such as indole acetic acid or kinetin, and the obtained cultured cell is then subjected to a reaction. At this time, a medium and culture conditions that are improved in order to enhance a phenylalanine ammonia-lyase activity may also be used.

For example, there are known some reports such as a report by Yazaki et al. regarding culture conditions for obtaining a cultured cell, into which the enzyme activity of *Lithospermum erythrorhizon* is induced (*Biosci. Biotech. Biochem.* (1997), 61(12), 1995-2003); a report by Klaus et al. regarding the relationship between the above enzyme activity and culture conditions in the cultured cell of *Petroselinum hortense (Archiv. Biochem. Biophis.* (1975), 66, 54-62); etc. Moreover, a ground product, a freeze-dried product or an extract from the thus obtained plant cultured cell; a product obtained by concentrating and extracting an ingredient catalyzing a reaction of interest from the extract; a product obtained by immobilizing the extract or extracted ingredient of the plant cultured cell on a hardly soluble carrier; etc., can also be subjected to a reaction.

In another embodiment of the present invention, the pal gene derived from a plant is introduced into a microorganism or plant so that the gene is expressible therein, and the resulting transformed microorganism or transformed plant can also be used. A host organism used for gene expression is not particularly limited, to the extent that the organism is known as an organism capable of uptaking and expressing an extraneous gene. Examples of such an organism include a bacterium such as *Escherichia, Pseudomonas* or *Bacillus,* yeast such as *Saccharomyces* or *Schizosaccharomyces,* and filamentous fungi such as *Aspergillus.*

The pal gene used in the present invention may be not only a pal gene naturally occurring in the environment, but also genes having the same functions (encoding mutant enzymes with the same activity). Examples of such a gene include a pa12 gene derived from *Lithospermum erythrorhizon* (Database Accession No. D83076); a pal gene encoding an amino acid sequence having 70% or more homology with the amino acid sequence deduced from the nucleotide sequence of the pal2 gene derived from *Lithospermum erythrorhizon;* or preferably, a pal gene encoding an amino acid sequence having 80% ormore homology with the amino acid sequence deduced from the nucleotide sequence of the pa12 gene derived from *Lithospermum erythrorhizon.* As stated above, phenylalanine ammonia-lyases derived from a plant have many commonalties in these functions and properties with one another, such as sufficiently high sequence homology of the plant pal genes and the identical functions in a plant body, and thus the equivalent effect can be expected in the present production method.

As for a method of isolating the cDNA of the enzyme gene from a plant and preparing a transformed microorganism by use of the gene, or a method of separating and collecting the enzyme from the transformed microorganism, there are known many reports such as a report by Yazaki et al. regarding a case of the use of *Lithospermum erythrorhizon (Biosci. Biotech. Biochem.* (1997), 61(12), 1995-2003), a report by W. Schulz et al. regarding a case of the use of *Petroselinum crispum (FEBS Letter* (1989), 258(2), 335-338), etc.

Next, a method of obtaining a pal gene, a method of preparing expressible plasmids, and a method of introducing these plasmids into various types of organisms and expressing them therein will be further specifically described.

### <Method of obtaining pal gene>

The pal gene derived from a plant can be obtained from a cDNA library produced by a known method, using a partial fragment corresponding to the conserved sequence of phenylalanine ammonia-lyase as a probe. After isolation and purification of conventional mRNA, necessary cDNA can be obtained with Reverse transcriptase, using the thus obtained mRNA as a template. In recent years, however, a sufficient amount of cDNA can easily be obtained by PCR method with heat-resistant Reverse transcriptase, using total RNA or mRNA as a template. The thus obtained cDNA is ligated to a suitable plasmid vector such as pBR322 or pUC18 to transform *Escherichia coli* used as a host.

To obtain the pal gene of interest from the cDNA library produced as above, there can be used a colony hybridization method wherein oligo DNA encoding the conserved sequence of phenylalanine ammonia-lyase is used as a probe. As stated above, since phenylalanine ammonia-lyase derived from a plant, over the entire sequence, has highly homologous sequences that are presumed as conserved sequences, oligo DNA used as a probe can be produced by selecting a position depending on a purpose and can be used.

A plasmid is prepared from a positive candidate transformant obtained by colony hybridization. Then, the plasmid is subjected to PCR in combination with suitable primers so as to confirm the position and direction of the pal gene which is inserted into the plasmid. The primary sequences of many pal genes derived from plants are disclosed in the database such as GenBank or EMBL. Accordingly, as for genes with sequence information, it is also possible to confirm the preparing and direction by preparing a restriction map.

### <Method of preparing expression plasmid and method of preparing recombinant microorganism used for production>

With a suitable vector (specific examples thereof will be described later), the pal gene is introduced into a bacterium such as *Escherichia coli* or a microorganism such as yeast including *Saccaromyces cerevisiae* and is expressed therein, so that a recombinant having an activity to generate a phenylalanine derivative of interest can be obtained. Herein, a preferable host is a bacterium such as *Escherichia coli.* As described later, the generated phenylalanine derivative can be isolated and purified by a conventional method of separating a reaction product of a microorganism from reaction mixture.

A method of preparing a plasmid containing an extraneous gene, and a procedure or method of introducing the plasmid into a microorganism such as *Escherichia coli* and expressing it, may be carried out according to a measure or method commonly used in the field of genetic engineering (see e.g. "Vectors for cloning genes", *Methods in Enzymology,* 216, pp.469-631, 1992, Academic Press, and "Other bacterial systems", *Methods in Enzymology,* 204, pp.305-636, 1991, Academic Press).

As a method of introducing extraneous genes (a group of pal genes) into *Escherichia coli,* there have already been established several efficient methods such as the Hanahan method or the rubidium method, and thus these method may be used for introduction of the genes (see e.g. Sambrook, J., Fritsch, E. F., Maniatis, T., "Molecular cloning-A laboratory manual." Cold Spring Harbor Laboratory Press, 1989). Expression of an extraneous gene in *Escherichia coli* may be carried out by a conventional method (see e.g. the above mentioned "Molecular cloning - A laboratory manual."), and a vector for *Escherichia coli* having a lac promoter or the like such as pUC, pBluescript, etc., can be used. Using a vector for *Escherichia coli*, pBluescript II SK- having a lac promoter, the present inventors inserted a pal gene downstream of the lac promoter and then allowed for the gene expression in *Escherichia coli.*

As a method of introducing a pal gene into yeast, *Saccharomyces cerevisiae,* there have already been established methods such as the lithium method, and introduction of the gene may be carried out using such a method (see e.g. "New Biotechnology of Yeast" under the editorship of Yuichi Akiyama, edited by Bioindustry Association, published by Igaku Syuppan Center). Using a promoter such as PGK or GPD (GAP) and a terminator, expression of an extraneous gene in yeast can be carried out by: constructing an expression cassette into which an extraneous gene is inserted between the promoter and the terminator to undergo transcription control; and inserting this expression cassette into a vector of *S. cerevisiae,* e.g. YRp (a multi-copy vector for yeast having, as a replication origin, the ARS sequence of the yeast chromosome), YEp (a multi-copy vector for yeast having the replication origin of 2 µm DNA of the yeast), YIp (a vector for incorporation of a yeast chromosome that does not have any replication origins of the yeast), etc. (see the above mentioned "New Biotechnology of Yeast" Igaku Syuppan Center, and "Genetic Engineering for Production of Substances", Japan Society for Bioscience, Biotechnology and Agrochemistry, ABC Series, published by Asakura Shoten).

### <Method of culturing recombinant microorganism>

Culturing the transformant of the present invention can be carried out according to a common method of culturing a host microorganism.

A carbon source for culturing a transformed microorganism can be a material that a host microorganism can use as a carbon source, and examples of such a carbon source include saccharides such as glucose, sucrose, fructose and blackstrap molasses; organic materials such as ethanol, acetic acid, citric acid, succinic acid, lactic acid, benzoic acid and fatty acid, or alkali metal salts thereof; aliphatic hydrocarbons such as n-paraffin; and natural organic materials such as peptone, meat extract, fish extract, soybean meal, wheat bran, malt extract and potato extract. These materials can be used singly or in combination at a concentration of usually around 0.01% to 30%, and preferably around 0.1% to 10%. Where *Escherichia coli* is used as a transformant, glucose, peptone, meat extract, malt extract, etc. can preferably be used.

A nitrogen source for culturing a transformed microorganism can be a material that a host microorganism can use as a nitrogen source, and examples of such a nitrogen source include inorganic nitrogen compounds such as ammonium sulfate, ammonium phosphate, sodium nitrate and potassium nitrate; nitrogen-containing organic materials such as urea and uric acid; and natural organic materials such as peptone, meat extract, fish extract, soybean meal, malt extract and potato extract. These materials can be used singly or in combination at a concentration of usually around 0.01% to 30%, and preferably around 0.1% to 10%. Where *Escherichia coli* is used as a transformant, ammonium sulfate, peptone, meat extract, malt extract, etc. can preferably be used.

Further, as necessary, phosphate such as potassium dihydrogen phosphate or a metal salt such as magnesium sulfate, ferrous sulfate, calcium acetate, manganese chloride, copper sulfate, zinc sulfate, cobalt sulfate or nickel sulfate is added to promote the growth of bacterial cells. The additive concentration depends on culture conditions, but in general, the concentration is about 0.01% to 5% for phosphate, about 10 ppm to 1% for magnesium salt, and about 0.1 ppm to 1,000 ppm for other compounds. Furthermore, depending on a medium to be selected, approximately 1 ppm to 100 ppm of yeast extract, casamino acid, yeast nucleic acid or the like can be added as a source of supplying vitamins, amino acids or nucleic acids to promote the growth of bacterial cells.

Still further, as necessary, an inducer corresponding to the expression promoter of a vector can be added during culture so as to allow the enzyme gene provided for transformation to highly express itself in a host microorganism. Where a lac promoter is used, about 0.01 mM to 10 mM of IPTG (isopropyl-1-thio-β-D-galactoside) is added, and where a promoter for resistance to a drug such as ampicillin or kanamycin is used, about 0.1 ppm to 1,000 ppm of the corresponding drug is added.

In all cases where any composition is used, pH is maintained at 5 to 9, and preferably 5.5 to 8 for culture. Moreover, the procedures in which a microorganism cell cultured in advance in the above described medium is separated and collected from a culture solution by a method such as centrifugation or membrane filtration and subjected to a reaction are useful to reduce impurities taken from the culture solution and to facilitate the subsequent separation and collection of a product.

The transformed microorganism obtained by culturing can be collected by a known method such as centrifugation or membrane filtration and used for a reaction of interest, otherwise the culture solution can directly be subjected to the reaction of interest. Furthermore, a ground or freeze-dried product of the transformed microorganism; a cell-free extract from the microorganism cell; a product obtained by concentrating and extracting an ingredient catalyzing a reaction of interest from the cell-free extract; a product obtained by immobilizing these transformed microorganism cells, and a treated product, an extract and an extracted ingredient thereof on a hardly soluble carrier; etc. can also be subjected to a reaction of interest.

Examples of an immobilizing carrier used for the above purpose include polyacrylic acid, polymethacrylic acid, polyacrylamide, polyvinyl alcohol, poly-N-vinylformamide, polyallylamine, polyethyleneimine, methylcellulose, glucomannan, alginate, carrageenan, etc., and the (crosslinked) copolymers of these compounds. In other words, a compound that forms a water-insoluble solid comprising a microorganism or an extract ingredient thereof can be used singly or in a mixed form. Moreover, a transformed microorganism, an extract or an extract ingredient thereof can be retained on a solid substance such as an activated carbon, porous ceramics, a glass fiber, a porous polymer molding or a nitrocellulose membrane before the use.

### <Method of preparing and culturing recombinant plant used for production>

As stated above, a plant generating a phenylalanine derivative can be prepared by introduction of a pal gene into the plant cell. As a preferred example, a plasmid containing the pal gene is prepared, and the plasmid is then introduced into cells of a suitable plant such as *Nicotiana tabacum* and allowed for expression to obtain a plant generating a phenylalanine derivative of interest. There have been published a lot of studies regarding techniques for introduction and expression of various types of genes in a plant, and a plant having an enhanced activity of interest can be obtained by these known methods (*PlantMol. Biol.* (1999), 39(4), 683-693, *Plant Biotechnol.* (Tokyo) (1998), 15(4), 189-193, *Plant Physiol.* (1996), 112(4), 1617-1624).

Examples of a known method of introducing an extraneous gene into a plant cell include a method involving a phytopathogenic bacterium *Agrobacterium tumefaciens,* the electroporation method, a method involving a particle gun, etc., and a suitable method can be selected from these methods depending on the type of a plant, into which the gene is intended to be introduced. As a promoter, not only the 35S promoter from cauliflower mosaic virus (CaMV), which is a promoter for systemic high expression, but also other promoters specifically expressed in various organs, can be used. A binary vector pBI121 containing the 35S promoter from CaMV, can be obtained from Clontech, and this vector is broadly used as a vector mediating *Agrobacterium tumefaciens.* It has already been shown that the pal gene can be introduced into a plant such as *Nicotiana tabacum* by using a particle gun and then the introduced pal gene is expressed and functions therein (*Shokubutsu Soshiki Baiyo* (1995), 12(2), 165-171).

Examples of a method of preparation of a plasmid containing an extraneous gene, and a procedure or method of introduction and expression of a plasmid in a plant (the cell of a leaf, a stem, a root, etc.) not only include methods shown in the present invention, but also include methods conventionally used in the field of genetic engineering, and thus the preparation, introduction and expression of a plasmid may be carried out according to those techniques or methods (see e.g. Isao Ishida, Norihiko Misawa, *Saibo Kogaku Jikken Sosa Nyumon (An Introduction to Experiments of Cell Technology),* Kodansha, 1992). A recombinant plant prepared as above can be used for plant cell culture according to common methods of plant cell culture stated as above.

The reaction of generating an L-amino acid in the present invention is carried out by: adding, as a reaction material, 1 ppm to 20%, preferably 10 ppm to 10% acrylic acid derivative, and 2M to 12M at final concentration of ammonia; controlling pH at 8.5 to 11, preferably 9 to 10.5; and performing a reaction for about 1 to 200 hours, in the presence of phenylalanine ammonia-lyase derived from a plant that is prepared by various known methods as described above, a plant treated product or cultured cell having the activity of the enzyme, the same enzyme produced by a microorganism transformed with the enzyme gene isolated from the plant, a transformed microorganism cell and a treated product thereof, the same enzyme produced from a plant transformed with the enzyme gene, or a transformed plant and a treated product thereof.

Examples of acid used to adjust pH include inorganic acid such as sulfuric acid, hydrochloric acid, phosphoric acid, boric acid or carbonic acid, organic acid such as formic acid, acetic acid or propionic acid, and salts thereof. The use of a volatile acid herein resultingly requires only separation and elimination of cells from a reaction solution, and it makes possible to omit a desalinization step and to easily separate and collect a product. A preferred acid used therefor is carbonic acid. Examples of carbonic acid in this case include also carbonic acid that is dissolved in aqueous solution as carbon dioxide by bubbling or the like and is then generated by dissociation. Further, these acids and ammonium salts can also be used as ammonium sources for a reaction solution. For the above stated reasons, it is preferable to use ammonium carbonate or ammonium hydrogen carbonate as a part or all of the ammonium source.

It is noted that dissolving the whole amount of an acrylic acid derivative added is not necessarily required but it is also possible to add a solvent, a surfactant or the like that improves solubility or dispersibility of the derivative in a reaction solution. Depending on consumption of a compound due to progression of the reaction, the compound may be added consecutively or intermittently. The concentration of the compound in a reaction solution in this case is not always within the range stated above.

An L-amino acid generated in a reaction solution is separated and collected by a known method such as centrifugation, membrane filtration, vacuum drying, distillation, solvent extraction, salting out, ion exchange or various types of chromatography, depending on the properties of the derivative in the reaction solution. An example of simply separating and collecting the L-amino acid therefrom is as follows. An enzyme, an enzyme treated product, a transformed microorganism or a treated product thereof, etc. is removed from a reaction solution by filtration, centrifugation, dialysis or the like, and thereafter solvent extraction is carried out or the reaction solution is acidified so that an unreacted starting material, acrylic acid derivative, is precipitated and the precipitate is removed.

The pH of the obtained supernatant is again adjusted to around the isoelectric point of an L-amino acid, and the precipitated derivative is collected in the same manner as above. Thus, a product can efficiently be collected from a reaction solution with high purity. In addition, it is also useful to remove in advance residual ammonia from a reaction solution by distillation or the like, and to increase the concentration by removal of water to enhance the yield of the product at the isoelectric point.

More conveniently, control of pH of a reaction solution is carried out with volatile acid. Where the conversion rate is relatively high, the product can be directly isolated as an ammonium salt of an L-phenylalanine derivative after separation of cells from the reaction solution. Where a large amount of starting material still remains, after separation of microorganisms, the reaction solution is subjected to acidification before solvent extraction, thereby removing water and acid base and isolating the product as an ammonium salt of an L-amino acid. Preferred examples of volatile acid suitable for such a method include carbonic acid and an ammonium salt thereof.

In some cases, depending on the properties of a reaction product, the product accumulates in a reaction solution and thereby the reaction rate is reduced. In this case, it is preferable to employ a method of adding aqueous ammonia, ammonia-containing physiological saline and a reaction buffer containing ammonia into a reaction solution, depending on the concentration of a product, to consecutively dilute the reaction solution. Moreover, there is another method of increasing the reaction rate, in which cells are separated and collected when the reaction rate is reduced, the supernatant is collected as a solution containing a product, and the collected cells are replaced into a solution containing reaction materials or suspension. These methods can be performed repeatedly to the extent that the ammonia-lyase activity of a microorganism is maintained.

A compound used as a starting material in the present invention is an acrylic acid derivative represented by the above described general formula (1) : 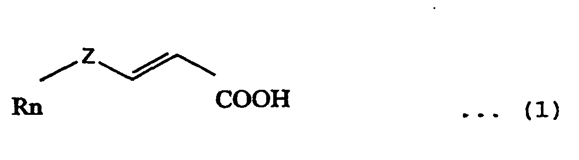 wherein
Z represents an aromatic ring group that may comprise a hetero atom,
R represents a substituent on the aromatic ring, and
n represents an integer of 0 or greater, and where n is 2 or greater, each R may be identical or different.

The obtained compound is a compound represented by the general formula (2) mentioned above: 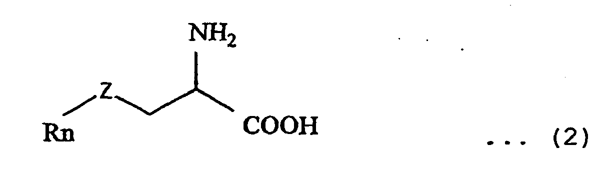 wherein
Z represents an aromatic ring group that may comprise a hetero atom,
R represents a substituent on the aromatic ring,
n represents an integer of 0 or greater, and where n is 2 or greater, each R may be identical or different.

In the above general formulas, examples of Z include: groups represented by the following general formulas (6) to (10) : 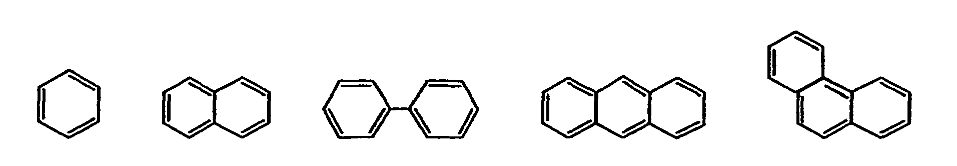 groups represented by the following general formulas (11) to (15) : 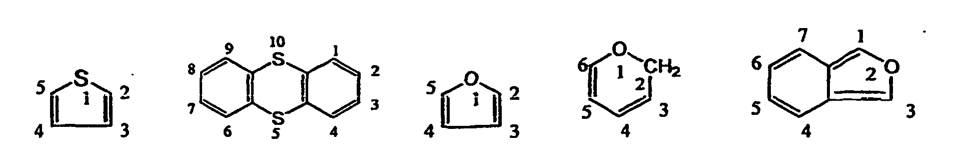 groups represented by the following general formulas (16) to (20) : 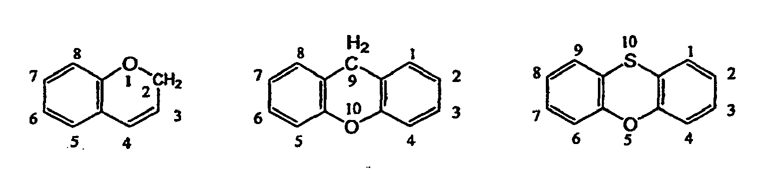 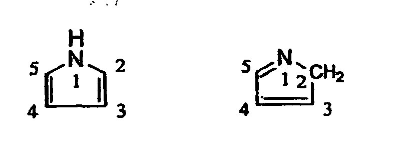 groups represented by the following general formulas (21) to (25) : 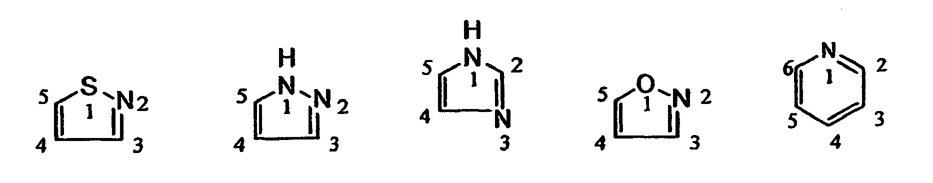 groups represented by the following general formulas (26) to (30) : 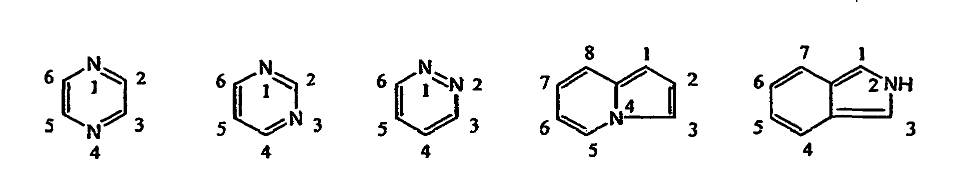 groups represented by the following general formulas (31) to (35) : 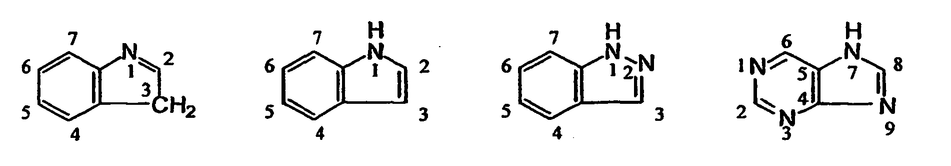 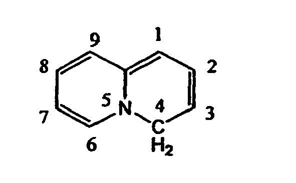 groups represented by the following general formulas (36) to (40) : 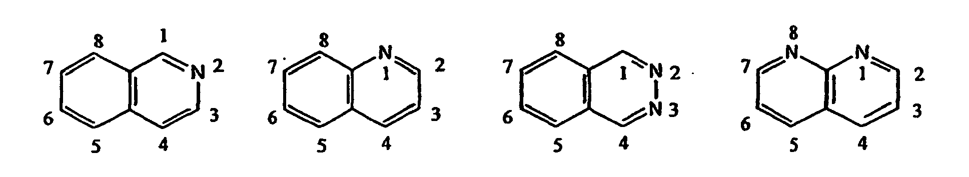 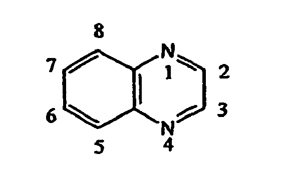 groups represented by the following general formulas (41) to (45) : 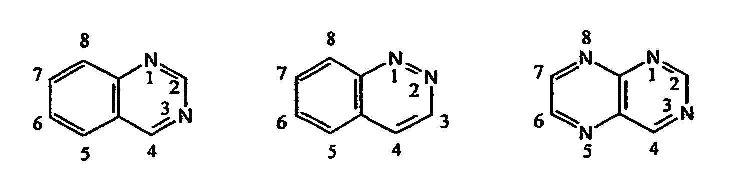 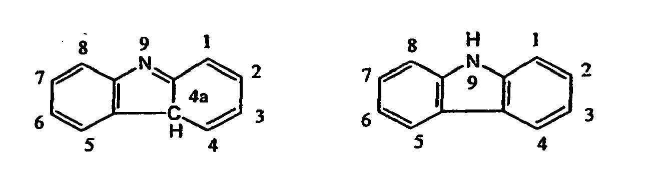 groups represented by the following general formulas (46) to (50) : 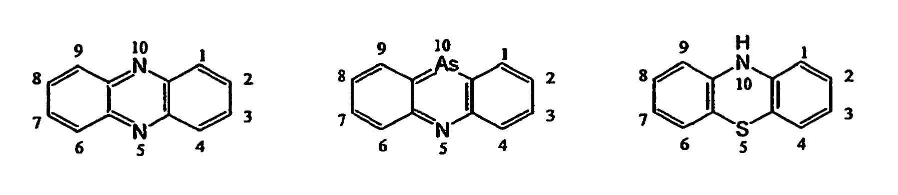 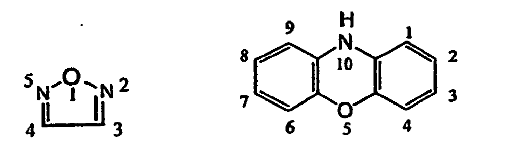 and, groups represented by the following general formulas (51) to (55) :

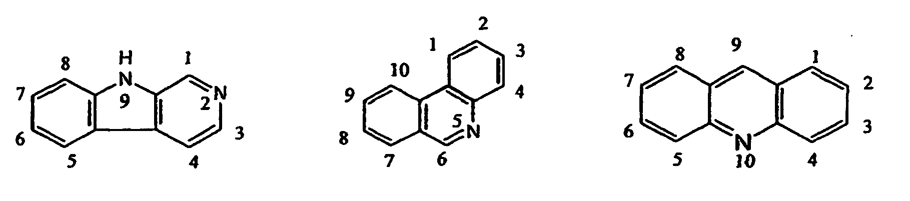

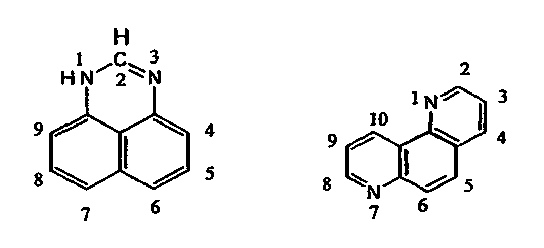

Examples of R in the above general formulas include a cyano group, hydroxyl group, carboxyl group, amide group, fluorine atom, chlorine atom, bromine atom, amino group, nitro group, hydroxymethyl group, or alkyl or alkoxy group having 1 to 6 carbon atoms.

In the above general formulas, n can be an integer of 0 or greater, which is a number obtained by subtracting 1 from the number of the position where an aromatic ring can be substituted, or smaller. Where n is 2 or greater, each R may be identical or different.

In the compound used as a starting material in the present invention represented by the above general formula (1), Rn-Z-is a compound represented by the following general formula (3): 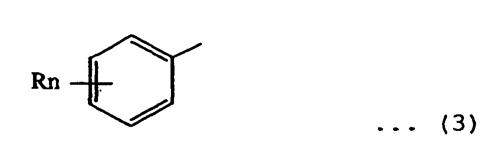 wherein
R is a substituent on a benzene ring and represents a cyano group, hydroxyl group, carboxyl group, amide group, fluorine atom, chlorine atom, bromine atom, amino group, nitro group, hydroxymethyl group, or alkyl or alkoxy group having 1 to 6 carbon atoms. n represents an integer of 0 to 5, and preferably an integer of 1 to 5. Where n is 2 or greater, each R may be identical or different.

The obtained compound is a compound, wherein, in the above general formula (2), Rn-Z- is represented by the above described general formula (4),
wherein R is a substituent on a benzene ring and represents a cyano group, hydroxyl group, carboxyl group, amide group, fluorine atom, chlorine atom, bromine atom, amino group, nitro group, hydroxymethyl group, or alkyl or alkoxy group having 1 to 6 carbon atoms. n represents an integer of 0 to 5, and preferably an integer of 1 to 5. Where n is 2 or greater, each R may be identical or different.

Specific examples of such a starting material include 2-cyanocinnamic acid, 3-cyanocinnamic acid, 4-cyanocinnamic acid, 2-hydroxycinnamic acid, 3-hydroxycinnamic acid, 4-hydroxycinnamic acid, 3,4-dihydroxycinnamic acid, 2-nitrocinnamic acid, 3-nitrocinnamic acid, 4-nitrocinnamic acid, 2-carboxycinnamic acid, 3-carboxycinnamic acid, 4-carboxycinnamic acid, 2-aminocinnamic acid, 3-aminocinnamic acid, 4-aminocinnamicacid, 2-chlorocinnamic acid, 3-chlorocinnamic acid, 4-chlorocinnamic acid, 2-fluorocinnamic acid, 3-fluorocinnamic acid, 4-fluorocinnamic acid, 2-bromocinnamic acid, 3-bromocinnamic acid, 4-bromocinnamic acid, 2-iodocinnamic acid, 3-iodocinnamic acid, 4-iodocinnamic acid, 2-methylcinnamic acid, 3-methylcinnamic acid, 4-methylcinnamic acid, 2-methoxycinnamic acid, 3-methoxycinnamic acid, 4-methoxycinnamic acid, 4-isopropylcinnamic acid, 4-t-butyl cinnamic acid, 4-methoxy-3-methylcinnamic acid, etc.

Where an enzyme derived from *Lithospermum erythrorhizon* is used, preferred starting materials to be used are 2-cyanocinnamic acid, 4-cyanocinnamic acid, 3-hydroxycinnamic acid, 4-hydroxycinnamic acid and 3, 4-dihydroxycinnamic acid. Where these starting materials are used, there can be obtained 2-cyano-L-phenylalanine, 4-cyano-L-phenylalanine, 3-hydroxy-L-phenylalanine (metatyrosine),
4-hydroxy-L-phenylalanine (tyrosine) and 3,4-dihydroxy-L-phenylalanine (DOPA), respectively. In the present invention, cinnamic acid derivatives include also cinnamic acid, and accordingly the corresponding phenylalanine derivatives include also phenylalanine.

Particularly, the compound used as a starting material in the present invention is a compound, wherein, in the above general formula (1), Rn-Z- is represented by the following general formula (4): 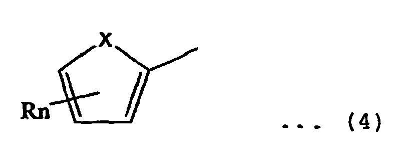 wherein
X represents S, O, NH or NR¹,
R¹ represents an alkyl group having 1 to 6 carbon atoms,
R is a substituent on a hetero ring and represents a cyano group, hydroxyl group, carboxyl group, amide group, fluorine atom, chlorine atom, bromine atom, amino group, nitro group, hydroxymethyl group, or alkyl or alkoxy group having 1 to 6 carbon atoms, and
n represents an integer of 0 to 3, and where n is 2 or greater, each R may be identical or different.

The obtained compound is a compound, wherein, in the above general formula (2), Rn-Z- is represented by the above general formula (4),
wherein
X represents S, O, NH or NR¹,
R¹ represents an alkyl group having 1 to 6 carbon atoms,
R is a substituent on a hetero ring and represents a cyano group, hydroxyl group, carboxyl group, amide group, fluorine atom, chlorine atom, bromine atom, amino group, nitro group, hydroxymethyl group, or alkyl or alkoxy group having 1 to 6 carbon atoms, and
n represents an integer of 0 to 3, and where n is 2 or greater, each R may be identical or different.

In the compound used as a starting material in the present invention represented by the above general formula (1), Rn-Z-is represented by the following general formula (5): 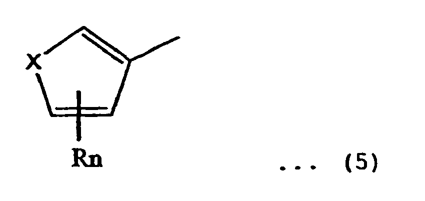 wherein
X represents S, O, NH or NR¹,
R¹ represents an alkyl group having 1 to 6 carbon atoms,
R is a substituent on a hetero ring and represents a cyano group, hydroxyl group, carboxyl group, amide group, fluorine atom, chlorine atom, bromine atom, amino group, nitro group, hydroxymethyl group, or alkyl or alkoxy group having 1 to 6 carbon atoms, and
n represents an integer of 0 to 3, and where n is 2 or greater, each R may be identical or different, and the obtained compound is a compound, wherein, in the above general formula (2), Rn-Z-is represented by the above general formula (5),
wherein
X represents S, O, NH or NR¹,
R¹ represents an alkyl group having 1 to 6 carbon atoms,
R is a substituent on a hetero ring and represents a cyano group, hydroxyl group, carboxyl group, amide group, fluorine atom, chlorine atom, bromine atom, amino group, nitro group, hydroxymethyl group, or alkyl or alkoxy group having 1 to 6 carbon atoms, and
n represents an integer of 0 to 3, and where n is 2 or greater, each R may be identical or different.

Specific examples of such a starting material include 2-furanacrylic acid, 3-furanacrylic acid, 2-thienyl acrylic acid, 3-thienyl acrylic acid, 2-pyrrolyl acrylic acid, 3-pyrrolyl acrylic acid, etc.

Where an enzyme derived from *Lithospermum erythrorhizon* is used, preferred starting materials to be used are 2-furanacrylic acid, 3-furanacrylic acid, 2-thienyl acrylic acid, 3-thienyl acrylic acid, 2-pyrrolyl acrylic acid and 3-pyrrolyl acrylic acid. Where these starting materials are used, there can be obtained alpha-amino-2-furanpropionic acid, alpha-amino-3-furanpropionic acid, alpha-amino-2-thienyl propionic acid, alpha-amino-3-thienyl-propionic acid, alpha-amino-2-pyrrole-propionic acid, and alpha-amino-3-pyrrole-propionic acid, respectively.

According to the method of the present invention, an L-amino acid having high optical purity can efficiently be obtained by a single step of reaction, using, as a substrate, an acrylic acid derivative that is conveniently obtained by organic synthesis. The thus obtained L-amino acid is useful as a synthesis intermediate in the field of fine chemicals such as pharmaceuticals and agricultural chemicals, which requires high optical purity.

### EXAMPLES

The present invention will be further specifically described in the following examples. The examples are provided for illustrative purposes only, and are not intended to limit the scope of the invention.

It should be noted that, in the following examples and comparative examples, instead of free acids, their salts or esters may be used as starting materials. And also "1 unit" is defined as the amount of enzyme which releases 1 µmole of cinnamic acid per minute at 30°C in the presence of 0.1M sodium borate buffer (pH 8.5) and 10 mML-phenylalanine as a substrate.

Enzyme protein amount is determined using a standard such as bovine serum albumin by the biuret method, and a specific activity of the enzyme is expressed in "unit/mg protein". Identification of an acrylic acid derivative and an amino acid was carried out by ¹H-NMR and ¹³C-NMR and by subj ecting a reaction solution to HPLC and making comparison on UV absorption intensity with a standard.

Separation and determination of the obtained acrylic acid derivative was carried out under the following analysis conditions.

### Reverse phase HPLC analysis conditions

Column: Shodex (Trade mark registered by Showa Denko K.K.), RSpak NN-614 (produced by Showa Denko K.K.)
Column temperature: 40°C
Eluent: acetonitrile/water/a 50 mM H₃PO₄-KH₂PO₄ solution (pH 3) = 20/70/10
Flow rate: 1.0 ml/min
Detection: by absorption of UV at 210 nm

Analysis of the optical purity of the obtained amino acid was carried out by optical resolution HPLC under the following conditions.

### Optical resolution HPLC analysis conditions

Column: Shodex (Trade mark registered by Showa Denko K.K.), ORpak CRX-853 (produced by Showa Denko K.K.)
Column temperature: room temperature (22°C)
Eluent: acetonitrile/water = 15/85 2.5 mM CuSO₄
Flow rate: 1.0 ml/min
Detection: by absorption of UV at 256 nm

### Example 1: Reaction by cultured cell (Petroselinum hortense)

The composition of medium A is shown in Table 1.

**[Table 1]**

| Ingredients | mg/L |
|---|---|
| Disodium phosphate | 150 |
| Potassium nitrate | 3000 |
| Ammonium sulfate | 134 |
| Magnesium sulfate heptahydrate | 500 |
| Calcium chloride dihydrate | 150 |
| Ferrous sulfate heptahydrate | 15 |
| Ethylenediaminetetraacetic acid | 15 |
| Nicotinic acid | 1 |
| Thiamine hydrochloride | 10 |
| Pyridoxine hydrochloride | 1 |
| m-inositol | 100 |
| Manganese sulfate hydrate | 10 |
| Boric acid | 3 |
| Zinc sulfate heptahydrate | 2 |
| Sodium molybdate dihydrate | 0.25 |
| Cupric sulfate | 0.025 |
| Cobalt chloride hexahydrate | 0.025 |
| Potassium iodide | 0.75 |
| Sucrose | 20000 |
| 2,4-dichlorophenoxyacetic acid (2,4-D) | 2 |
| pH = 5.5 | |

Ten L of medium A having the above described composition was dividedly poured into Erlenmeyer flasks, and then a parsley (*Petroselinum hortense*) cell culture suspension which had previously been subcultured in the same medium at 27°C in the dark under aeration (the 5^{th} generation of subculture) was inoculated into each medium followed by shaking culture at 27°C in the dark under aeration at 200 rpm for 10 days. Then, the culture was further continued for 24 hours while the light of a fluorescent lamp was applied thereto. The obtained culture solution was subjected to vacuum filtration with a glass fiber filter to obtain about 1.6 kg of cultured cells containing phenylalanine ammonia-lyase. The activity was 0.0051 u/g per fresh cell weight.

Two g of the obtained cultured cells was suspended in 10 mL of a 2M ammonia/ammonium carbonate solution (pH 10) (which was obtained by adjusting pH by mixing a 2M ammonia water and a 2M ammonium carbonate solution) containing 0.1% of various acrylic acid derivatives, and a reaction was carried out by stirring at 30°C for 24 hours. Table 2 shows products, and concentrations and optical purities thereof, which were obtained by each reaction.

### Comparative example 1: Reaction by enzyme derived from microorganism

A reaction was carried out under the same conditions as in Example 1, with the only exception that 0.025 mg of commercially available phenylalanine ammonia-lyase derived from *Rhodotorula glutinis* (Sigma) having a specific activity of 0.44 unit/mg was added instead of the cultured cells in Example 1. The obtained products, and concentrations and optical purities thereof are shown in Table 2.

**[Table 2]**

| | | | | | | |
|---|---|---|---|---|---|---|
| Substrates | | Cinnamic acid | 3-hydroxycinnamic acid | 3,4-dihydroxycinnamic acid | 2-cyanocinnamic acid | 4-cyanocinnamic acid |
| Products | | L-phenylalanine | Metatyrosine | L-DOPA | L-2-cyanophenylalanine | L-4-cyanophenylalanine |
| Product concentration (mg/L) | Example 1 | 210 | 180 | 170 | 240 | 250 |
| | Comparative example 1 | 190 | 50 | 30 | 5 | Non-detected |
| Product optical purity (%) | Example 1 | >99.9 | >99.9 | >99.9 | >99.9 | >99.9 |
| | Comparative example 1 | >99.9 | >99.9 | >99.9 | >99.9 | - |

### Example 2: Reaction by treated product of cultured cell (Petroselinum hortense)

The *Petroselinum hortense* cultured cells (1.5 kg) obtained by culturing in the same manner as in Example 1 was suspended in 3 L of a 0.1M sodium borate buffer solution (pH 8.8), and the suspension was subjected to ultrasonic homogenization on ice for 20 minutes. An insoluble fraction was settled out from the obtained treated product by centrifugation at 10,000 rpm for 15 minutes, and the supernatant was collected.

Crushed ammonium sulfate was gradually added to the obtained extract on ice over 30 minutes, while stirring, so that the concentration became 40% saturation, and then the solution was subjected to centrifugation at 10,000 rpm for 15 minutes to remove the precipitate. Likewise, ammonium sulfate was added to the obtained supernatant, so that the concentration became 55% saturation, and then the solution was subjected to centrifugation at 10, 000 rpm for 15 minutes to collect the precipitate. The obtained precipitate was dissolved in 150 ml of 0.05M Tris-HCl buffer solution, and then dialysis was carried out at 4°C for 24 hours against 100 times volume of the same buffer solution. After dialysis, 170 ml of the solution was obtained as a crude enzyme extract solution of phenylalanine ammonia-lyase. The total amount of protein was 620 mg, and the specific activity was 0.0205 U/mg.

To a mixed solution of 1 ml of the obtained crude extract solution and 9 ml of 2M ammonia/ammonium carbonate solution (pH 10) (which was obtained by adjusting pH by mixing a 2M aqueous ammonia and a 2M ammonium carbonate solution), various cinnamic acid derivatives were added, so that the mixed solution contained 0.5% of the derivatives, and a reaction was then carried out by stirring at 30°C for 24 hours. Table 3 shows products, and concentrations and optical purities thereof, which were obtained by each reaction.

### Comparative example 2: Reaction by enzyme derived from microorganism

A reaction was carried out under the same conditions as in Example 2, with the only exception that 0.175 mg of commercially available phenylalanine ammonia-lyase derived from *Rhodotorula graminis* (Sigma) having a specific activity of 0.44 unit/mg was added instead of the crude enzyme extract solution of Example 2. The obtained products, and concentrations and optical purities thereof are shown in Table 3.

**[Table 3]**

| | | | | | | |
|---|---|---|---|---|---|---|
| Substrates | | Cinnamic acid | 3-hydroxycinnamic acid | 3,4-dihydroxycinnamic acid | 2-cyanocinnamic acid | 4-cyanocinnamic acid |
| Products | | L-phenylalanine | Metatyrosine | L-DOPA | L-2-cyanophenylalanine | L-4-cyanophenylalanine |
| Product concentration (mg/L) | Example 2 | 1920 | 1600 | 1500 | 2010 | 2040 |
| | Comparative example 2 | 1680 | 470 | 200 | 40 | Non-detected |
| Product optical purity (%) | Example 2 | >99.9 | >99.9 | >99.9 | >99.9 | >99.9 |
| | Comparative example 2 | >99.9 | >99.9 | >99.9 | >99.9 | - |

### Example 3: Reaction by treated product of plant tissues (Cucumis sativus L.; cucumber)

Cucumber (*Cucumis sativus* L.) seeds were cultured in a petri dish, on which a water-wet filter was placed, at 25°C for 72 hours for germination. Then, the light of a fluorescent lamp was applied thereto for 4 hours, and thereafter about 50 g of hypocotyls was collected.

The obtained hypocotyl tissues were homogenized in a mortar on ice, and then the total amount of the homogenized product was suspended in 100 ml of 0.1M sodium borate buffer solution (pH 8.8) containing 1 mM glutathione followed by ultrasonic homogenization for 20 minutes. An insoluble fraction was settled out from the obtained treated product by centrifugation at 10,000 rpm for 15 minutes, and the supernatant was collected.

Crushed ammonium sulfate was gradually added to the obtained extract on ice over 30 minutes, while stirring, so that the concentration became 30% saturation, and then the solution was subjected to centrifugation at 10,000 rpm for 15 minutes to remove the precipitate. Likewise, ammonium sulfate was added to the obtained supernatant, so that the concentration became 70% saturation, and then the solution was subjected to centrifugation at 10, 000 rpm for 15 minutes to collect the precipitate. The obtained precipitate was dissolved in 20 ml of 0.05M Tris-HCl buffer solution, and then dialysis was carried out at 4°C for 24 hours against 100 times volume of the same buffer solution. After dialysis, 2.2 ml of the solution was obtained as a crude enzyme extract solution. The total amount of protein was 50.7 mg, and the specific activity was 0.0307 U/mg.

To a mixed solution of 0.1 ml of the obtained crude extract solution and 9. 9 ml of 2M ammonia/ammonium carbonate solution (pH 10) (which was obtained by adjusting pH by mixing a 2M aqueous ammonia and a 2M ammonium carbonate solution), various cinnamic acid derivatives were added so that the mixed solution contained 0.5% of the derivatives, and a reaction was then carried out by stirring at 30°C for 24 hours. Table 4 shows products, and concentrations and optical purities thereof, which were obtained by each reaction.

### Comparative example 3: Reaction by enzyme derived from microorganism

As a comparative example, a reaction was carried out under the same conditions as in Example 3, with the only exception that 0.16 mg of commercially available phenylalanine ammonia-lyase derived from *Rhodotorula graminis* (Sigma) having a specific activity of 0.44 unit/mg was added instead of the crude enzyme extract solution of Example 3. The obtained products, and concentrations and optical purities thereof are shown in Table 4.

**[Table 4]**

| | | | | | | |
|---|---|---|---|---|---|---|
| Substrates | | Cinnamic acid | 3-hydroxycinnamic acid | 3,4-dihydroxycinnamic acid | 2-cyanocinnamic acid | 4-cyanocinnamic acid |
| Products | | L-phenylalanine | Metatyrosine | L-DOPA | L-2-cyanophenylalanine | L-4-cyanophenylalanine |
| Product concentration (mg/L) | Example 3 | 1480 | 1300 | 1280 | 1940 | 2040 |
| | Comparative example 3 | 1500 | 400 | 180 | 40 | Non-detected |
| Product optical purity (%) | Example 3 | >99.9 | >99.9 | >99.9 | >99.9 | >99.9 |
| | Comparative example 3 | >99.9 | >99.9 | >99.9 | >99.9 | - |

### Example 4: Culture of transformant and reaction with transformant

Cells were cultured for 24 hours on an agar plate that was obtained by mixing 2% agar to an L broth (1% polypeptone, 0.5% NaCl, 0.5% yeast extract, pH 7.0) and flattening the mixture. Thereafter, an inoculating loop amount of the cell was further cultured with 5 ml to 100 ml of L broth at 30°C for 16 hours to obtain a transformant used for a reaction. After culture, the cells were collected and washed with the same volume of physiological salt solution as the culture solution. Then, the cell bodies were suspended in a reaction solution of a half volume of the culture solution (4M ammonia/ammonium carbonate (pH 10.3)), and thereto a substrate was further addedtobe 0.2% (2,000mg/L) at final concentration followed by a reaction at 30°C while stirring. An aliquot of this reaction solution was taken every certain hours, cells were removed by centrifugation, and the supernatant was subj ected to HPLC analysis to determine the amount of a product.

### Example 5: Preparation of cDNA library and acquisition of pal gene

Cell culture of murasaki (*Lithospermum erythrorhizon*), preparation of the cDNA library and acquisition of the pal gene are described in detail in the report by Yazaki et al. *(Plant Cell Physiol.* (1995), 36, 1319-1329; Biosci. *Biotech. Biochem.* (1997), 61(12), 1995-2003), and preparation of the cDNA library of tea (*Camellia sinensis)* and acquisition of the pal gene are described in detail in the report by Matsumoto et *al.* (*Theor. Appl. Genet.* (1994), 89(6), 671-675). With reference to the existing information, the present inventors have added some modification thereto and obtained the pal gene from each plant. A series of approaches are applicable to the pal genes from all plants, the nucleotide sequence of which has been clarified. In addition to cultured cells, plant tissues under conditions where the pal gene is expressed are also available as materials for obtaining mRNA.

### (Preparation of cDNA library of Lithospermum erythrorhizon)

Cells prepared from the tissues of *Lithospermum erythrorhizon* were cultured in an LS medium (a conventional growth medium containing 10 µM indoleacetic acid and 0.1 µM kinetin) at 25°C for 1 week. The obtained cultured cells were then transferred to an M9 medium (a shikonin growth medium containing 10 µM indoleacetic acid and 0.1 µM kinetin) and further cultured for 2 days. Using a QuickPrep Micro mRNA Purification Kit (Amersham Pharmacia Biotech), mRNA was prepared from the obtained cultured cells according to a standard method in the kit instructions. Subsequently, using a First-Strand cDNA Synthesis Kit (Amersham Pharmacia Biotech) and Oligo (dT) 12-18 primers, RT-PCR was carried out with the obtained mRNA as a template. A mixture of the generated DNA fragments was provided as a cDNA library used as a template for gene amplification PCR.

### (Preparation of cDNA library of Camellia sinensis)

DNA fragments were obtained from frozen young leaves of *Camellia sinensis* (collected in the afternoon of good sunshine) by RT-PCR in the same manner as for the above *Lithospermum erythrorhizon,* and a mixture of the obtained DNA fragments was used as a cDNA library used as a template for gene amplification PCR.

### (Acquisition of pal gene)

There exist two types of pal genes in *Lithospermum erythrorhizon* (hereinafter, referred to as "LePAL1" and "LePAL2"). The two types of the genes have already been published by Yazaki et al. in the databases such as GenBank and EMBL as SEQ ID NOS: D83075 and D83076, respectively. A pal gene existing in *Camellia sinensis* (hereinafter, referred to as "CAMPAL") has already been published by Matsumoto et al. in the above databases as SEQ ID NO: D26596. Using the sequences of 5'-terminus and 3'-terminus thereof, primers specific for the pal genes were prepared. Herein, primers were designed so as to have suitable restriction sites at both ends, so that the obtained DNA fragment can be ligated to a plasmid vector.

As a result of searching suitable restriction enzymes (which had no cleavage sites in the sequence of the pal gene) from among restriction enzymes that were often used for the multi-cloning sites of various types of plasmid vectors, it was determined that, as for LePAL2, an EcoRI site was used at a 5'-terminal side thereof and a SalI site was used at a 3'-terminal side thereof, and as for CAMPAL, a SmaI site was used at a 5' -terminal side thereof and a HindIII site was used at a 3'-terminal side thereof. Since there were found no sites suitable for the 5'-terminal side of LePAL1, an EcoRV site was used at the 5'-terminus, and a blunt end generated after cleavage was designed to ligate to the blunt end of the SmaI site of a vector. As for the 3'-terminal side of LePAL1, a SalI site was used just as with LePAL2.

### Sequences of primers:

(LePAL1)

### [Sequence 1]

5'-terminus (with an EcoRV site) : A23meroligonucleotide which has a sequence shown in Sequence Listing 1

### [Sequence 2]

3' -terminus (with a SalI site) : A 23mer oligonucleotide which has a sequence shown in Sequence Listing 2

(LePAL2)

### [Sequence 3]

5'-terminus (with an EcoRI site) : A 25mer oligonucleotide which has a sequence shown in Sequence Listing 3

### [Sequence 4]

3'-terminus (with a SalI site) : A 23mer oligonucleotide which has a sequence shown in Sequence Listing 4

(CAMPAL)

### [Sequence 5]

5' -terminus (with a SmaI site) : A 23mer oligonucleotide which has a sequence shown in Sequence Listing 5

### [Sequence 6]

3'-terminus (with a HindIII site): A 23mer oligonucleotide which has a sequence shown in Sequence Listing 6

### Composition of a reaction solution:

| | |
|---|---|
| Template cDNA | 0.5 to 2 µg |
| Primers | 100 pmol each |
| dNTPs solution | 1 mM each |
| 10 x reaction buffer | 10 µl |
| ExTaq DNA polymerase (TaKaRa Shuzo Co., Ltd.) | 2.5 U |
| Total: | 50 µl |

### Reaction conditions:

| | |
|---|---|
| Heat denaturation | 94°C, 30 seconds |
| Annealing | 55°C, 60 seconds |
| Elongation | 72°C, 120 seconds |
| Number of cycles | 24 cycles |

When amplification of gene fragments was attempted under the above conditions, while varying the amount of a template cDNA several times, a fragment having a length of about 2.1 kb of each of LePAL1, LePAL2 and CAMPAL, which was theoretically calculated, was almost specifically amplified. The thus amplified fragment of each of LePAL1, LePAL2 and CAMPAL was subj ected to agarose gel electrophoresis, and then extracted, collected and purified. Subsequently, when analysis of the nucleotide sequences was carried out with primers used for amplification, it was confirmed that the obtained information of the nucleotide sequences was matched with that of the sequences of LePAL1, LePAL2 and CAMPAL.

### Example 6: Preparation of transformant showing PAL activity

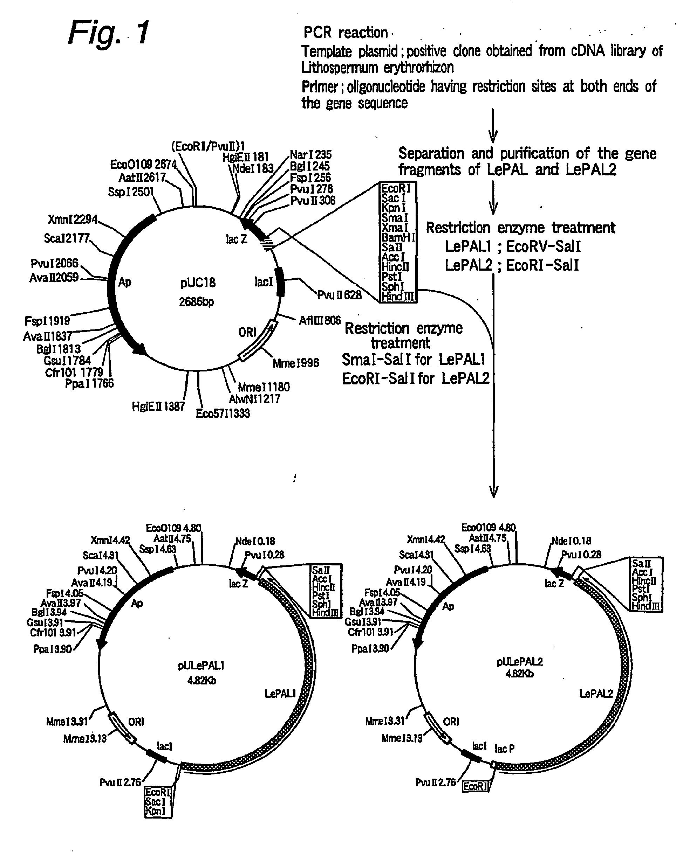

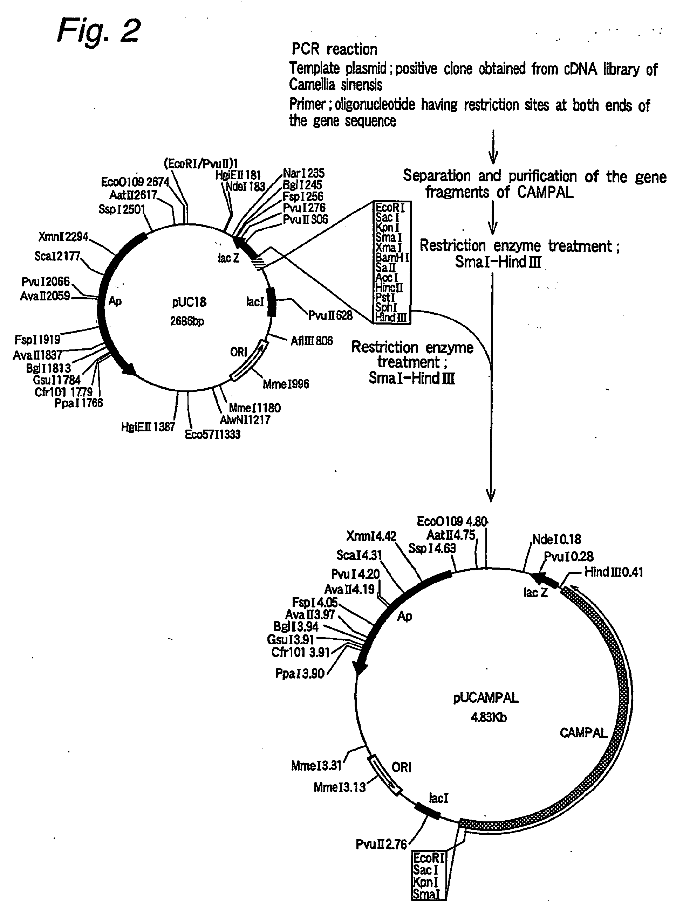

The obtained gene fragments were subjected to agarose gel electrophoresis followed by extraction and collection. The thus obtained fragments of LePAL1, LePAL2 and CAMPAL with restriction sites, were cleaved with restriction enzymes, EcoRV and SalI, EcoRI and SalI, and SmaI and HindIII, respectively. After that, operations such as agarose gel electrophoresis, extraction and collection were carried out again. These fragments were ligated to pUC18, which had been obtained by cleaving with EcoRV and SalI, EcoRI and SalI, and SmaI and HindIII, and then subjected to agarose gel electrophoresis, extraction and collection (FIGS. 1 and 2) .

The transformants obtained by transforming *Escherichia coli* JM109 with these plasmids were applied onto an L agar plate containing 0.1 mM isopropyl-β-D-thiogalactopyranoside (IPTG) and 0.1% X-Gal followed by culture at 37°C for 24 hours. From the generated colonies, some colonies presenting white color were selected, and the plamids thereof were prepared to examine the restriction enzyme cleavage patterns. As a result, a plurality of transformants having plasmids of interest, pULePAL1, pULePAL2 and pUCAMPAL, were obtained.

Three strains were arbitrarily selected from each type of the obtained transformants. According to the method described in Example 4, duplicates of each strain (2 species x 3 strains x 2 lines) were cultured in 5 ml of L broth. Twelve hours after initiation of the culture, isopropyl-β-D-thiogalactopyranoside (IPTG) was added to either one of the 2 lines, so that the IPTG concentration became 0.1 mM in the culture solution, followed by culture for 4 more hours. The obtained culture solutions were separately subjected to centrifugation to collect cells, and the obtained cells were subjected to a reaction according to the method described in Example 4. After 10 hours of the reaction, the obtained product was determined by HPLC. As shown in Table 5, regardless of the presence or absence of isopropyl-β-D-thiogalactopyranoside (IPTG), each transformant showed a phenylalanine ammonia-lyase activity.

**[Table 5]**

| | Activity in the absence of inducer, IPTG (mg/L) | Activity in the presence of inducer, IPTG (mg/L) |
|---|---|---|
| *Escherichia coli* JM109 | - | - |
| pULePAL1 transformant | 1960 | 1470 |
| pULePAL2 transformant | 1990 | 1830 |
| pUCAMPAL transformant | 1860 | 1520 |

### Example 7: Production of phenylalanine derivative using transformant

One strain from each type of transformants having pULePAL1, pULePAL2 and pUCAMPAL obtained in Example 6, was cultured in 100 ml of L broth containing 100 ppm ampicillin according to the method described in Example 4. The obtained culture solution was transferred to a 5 L jar fermenter which contains 2 L of L broth containing 100 ppm ampicillin, and then a stirring culture was carried out at 30°C at 800 rpm under aeration of 1 ml/min for 10 to 12 hours.

Cells obtained by centrifuging the culture solution from late logarithmic growth phase to early stationary phase were resuspended in 1 L of 4M ammonia/ammonium carbonate solution (pH 10.3), and 20 g of substrate (see Table 6) was added thereto followed by a reaction at 30°C while stirring at 800 rpm. An aliquot of the reaction solution was collected every one hour, and the product generated in the reaction solution (see Table 6) was determined by HPLC. While a substrate was successively added so that the concentration of the substrate was kept at about 2%, the reaction was continued. After about 10 hours, the product was accumulated at a concentration of 1% to 3% in the reaction solution (Table 6).

After completion of the reaction, the reaction solution was subj ected to vacuum concentration using a rotary evaporator to remove excessive ammonia, and concentrated hydrochloric acid was then added thereto to adjust pH at 1 to precipitate the substrate remained. The solution was filtrated with a filter, and then the supernatant was concentrated with a rotary evaporator. The product crystallized was collected by filtration and washed with diluted hydrochloric acid (0.1 N) followed by vacuum drying. The purity of this dried sample was 99% or more. A detected impurity was a cinnamic acid derivative or an acrylic acid derivative as a substrate in any system. When the optical purity of these derivatives was determined by the above described method, only the L form was detected in both derivatives and the D form was below the detection limit.

**[Table 6]**

| | | | | | | | |
|---|---|---|---|---|---|---|---|
| Substrates | | Cinnamic acid | 3-hydroxycinnamic acid | 3,4-dihydroxycinnamic acid | 2-cyanocinnamic acid | 4-cyanocinnamic acid | 2-furanacrylic acid |
| Products | | L-phenylalanine | Metatyrosine | L-DOPA | L-2-cyanophenylalanine | L-4-cyanophenylalanine | α-amino-2-furanpropionic acid |
| Product concentration (mg/L) | pULePAL1 transformant | 19200 | 12100 | 12000 | 20100 | 20400 | 11300 |
| | pULePAL2 transformant | 16800 | 13000 | 9800 | 21700 | 24900 | 11000 |
| | pUCAMPAL transformant | 15800 | 14700 | 10500 | 18600 | 22600 | 12500 |
| Product optical purity (%) | pULePAL1 transformant | >99.9 | >99.9 | >99.9 | >99.9 | >99.9 | >99.9 |
| | pULePAL2 transformant | >99.9 | >99.9 | >99.9 | >99.9 | >99.9 | >99.9 |
| | pUCAMPAL transformant | >99.9 | >99.9 | >99.9 | >99.9 | >99.9 | >99.9 |

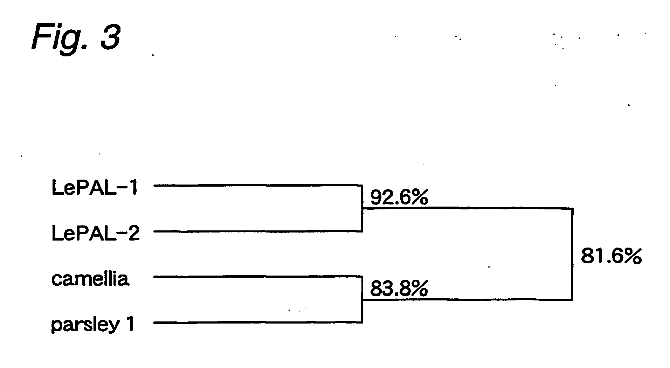

In the present examples, a plurality of phenylalanine ammonia-lyases having different amino acid sequences were employed to show that phenylalanine ammonia-lyase derived from a plant is generally effective in the present invention (the amino acid sequence of *Cucumis sativus* L. is unidentified) . The sequence homology in the amino acid sequences is shown in FIG. 3. It was found that any of these enzymes is sufficiently effective in the present invention although these enzymes are mutually 10% to 20% different in the amino acid sequences, that is, these enzymes have a much higher ability of converting substrates to generate a phenylalanine derivative than the same enzyme derived from a microorganism.

According to the present invention, by using phenylalanine ammonia-lyase derived from a plant, from an acrylic acid derivative as a starting material, the corresponding amino acid can conveniently and efficiently be obtained. In particular, an L-phenylalanine derivative having various substituents on a phenyl group thereof can simply and efficiently be obtained.

The L-amino acid obtained by the method for producing an L-amino acid of the present invention is useful as a chiral building block of a biologically active organic compound such as pharmaceutical intermediate or agricultural intermediate in the broad field, and it is useful mainly as pharmaceutical intermediate.

## Claims

1. A method for producing an L-amino acid, which comprises, in the presence of ammonia, allowing phenylalanine ammonia-lyase derived from a plant to act on an acrylic acid derivative represented by the following general formula (1) : 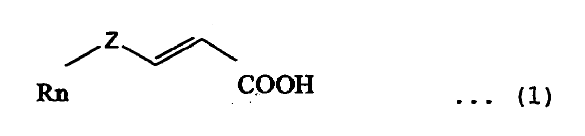 wherein
Z represents an aromatic ring group that may comprise a hetero atom,
R represents a substituent on said aromatic ring, and
n represents an integer of 0 or greater, and where n is 2 or greater, each R may be identical or different;
said L-amino acid being represented by the following general formula (2): 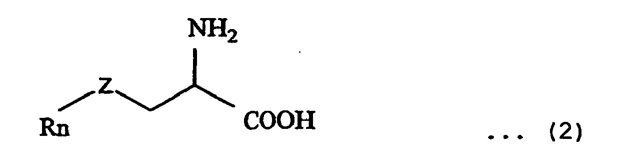 wherein
Z represents an aromatic ring group that may comprise a hetero atom,
R represents a substituent on said aromatic ring,
n represents an integer of 0 or greater, and where n is 2 or greater, each R may be identical or different.

2. The method for producing an L-amino acid according to claim 1, wherein Rn-Z- is represented by the following general formula (3): 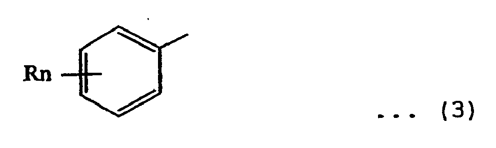 wherein
R is a substituent on a benzene ring and represents a cyano group, hydroxyl group, carboxyl group, amide group, fluorine atom, chlorine atom, bromine atom, amino group, nitro group, hydroxymethyl group, or alkyl or alkoxy group having 1 to 6 carbon atoms, and
n represents an integer of 0 to 5, and where n is 2 or greater, each R may be identical or different.

3. The method for producing an Z-amino acid according to claim 1, wherein Rn-Z- is represented by the following general formula (4): 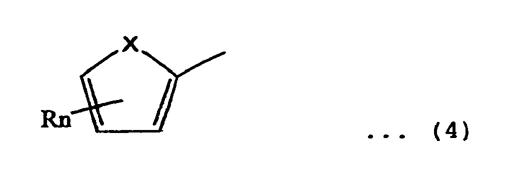 wherein
X represents S, O, NH or NR¹,
R¹ represents an alkyl group having 1 to 6 carbon atoms,
R is a substituent on a hetero ring and represents a cyano group, hydroxyl group, carboxyl group, amide group, fluorine atom, chlorine atom, bromine atom, amino group, nitro group, hydroxymethyl group, or alkyl or alkoxy group having 1 to 6 carbon atoms, and
n represents an integer of 0 to 3, and where n is 2 or greater, each R may be identical or different.

4. The method for producing an L-amino acid according to claim 1, wherein Rn-Z- is represented by the following general formula (5): 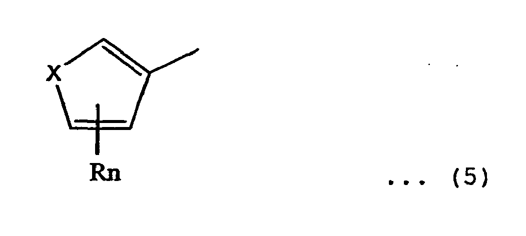 wherein
X represents S, O, NH or NR¹,
R¹ represents an alkyl group having 1 to 6 carbon atoms,
R is a substituent on a hetero ring and represents a cyano group, hydroxyl group, carboxyl group, amide group, fluorine atom, chlorine atom, bromine atom, amino group, nitro group, hydroxymethyl group, or alkyl or alkoxy group having 1 to 6 carbon atoms, and
n represents an integer of 0 to 3, and where n is 2 or greater, each R may be identical or different.

5. The method for producing an L-amino acid according to any one of claims 1 to 4, which comprises using a composition obtained by treating plant tissues containing a phenylalanine ammonia-lyase.

6. The method for producing an L-amino acid according to any one of claims 1 to 4, which comprises using a plant cultured cell containing a phenylalanine ammonia-lyase and/or a treated composition thereof.

7. The method for producing an L-amino acid according to any one of claims 1 to 4, which comprises allowing a phenylalanine ammonia-lyase gene derived from a plant to be expressible in a plant and using transformed plant cultures or a treated product of the plant cultures.

8. The method for producing an L-amino acid according to any one of claims 1 to 4, which comprises allowing a phenylalanine ammonia-lyase gene derived from a plant to be expressible in a microorganism and using a transformed microorganism culture, a treated product thereof, or an enzyme obtained from the culture.

9. The method for producing an L-amino acid according to claim 7 or 8, wherein the phenylalanine ammonia-lyase gene derived from a plant is a gene encoding an amino acid sequence having 70% or more homology with the amino acid sequence from deduced the nucleotide sequence of pal2 gene derived from *Lithospermum erythrorhizon.*

10. The method for producing an L-amino acid according to claim 7 or 8, wherein the phenylalanine ammonia-lyase gene derived from a plant is a gene encoding an amino acid sequence having 80% or more homology with the amino acid sequence deduced from the nucleotide sequence of pa12 gene derived from *Lithospermum erythrorhizon.*

11. The method for producing an L-amino acid according to any one of claims 1 to 8, wherein the plant from which the phenylalanine ammonia-lyase gene is derived is *Lithospermum* and/or *Camellia.*

12. The method for producing an L-amino acid according to any one of claims 8 to 11, wherein the microorganism is a bacterium, yeast and/or filamentous fungi.

13. The method for producing an L-amino acid according to claim 12, wherein the bacterium is *Escherichia coli.*

14. The method for producing an L-amino acid according to any one of claims 2 and 5 to 13, wherein, in said general formula (3), at least one of substituents R is a hydroxyl group.

15. The method for producing an L-amino acid according to any one of claims 2 and 5 to 13, wherein, in said general formula (3), at least one of substituents R is a cyano group.

16. The method for producing an L-amino acid according to any one of claims 2 and 5 to 13, wherein, in said general formula (3), at least one of substituents R is a carboxyl group.

17. The method for producing an L-amino acid according to any one of claims 2 and 5 to 13, wherein, in said general formula (3), at least one of substituents R is an amide group.

18. The method for producing an L-amino acid according to any one of claims 2 and 5 to 13, wherein, in said general formula (3), at least one of substituents R is a halogen group.

19. The method for producing an L-amino acid according to any one of claims 2 and 5 to 13, wherein, in said general formula (3), at least one of substituents R is an amino group.

20. The method for producing an L-amino acid according to any one of claims 2 and 5 to 13, wherein, in said general formula (3), at least one of substituents R is a nitro group.

21. The method for producing an L-amino acid according to any one of claims 2 and 5 to 13, wherein, in said general formula (3), at least one of substituents R is a hydroxymethyl group.

22. The method for producing an L-amino acid according to any one of claims 2 and 5 to 13, wherein, in said general formula (3), at least one of substituents R is an alkyl group having 1 to 6 carbon atoms.

23. The method for producing an L-amino acid according to any one of claims 1 to 13, wherein R is any one of a cyano group, a hydroxyl group, a nitro group and a carboxyl group, and n is 1.

24. The method for producing an L-amino acid according to any one of claims 2 and 5 to 13, wherein the L-amino acid is L-phenylalanine.

25. The method for producing an L-amino acid according to any one of claims 3 and 5 to 13, wherein, in said general formula (4), X is O.

26. The method for producing an L-amino acid according to any one of claims 3 and 5 to 13, wherein, in said general formula (4), X is S.

27. The method for producing an L-amino acid according to any one of claims 3 and 5 to 13, wherein, in said general formula (4), X is NH.

28. The method for producing an L-amino acid according to any one of claims 4 to 13, wherein, in said general formula (5), X is O.

29. The method for producing an L-amino acid according to any one of claims 4 to 13, wherein, in said general formula (5), X is S.

30. The method for producing an L-amino acid according to any one of claims 4 to 13, wherein, in said general formula (5), X is NH.

## Patentansprüche

1. Verfahren zur Herstellung einer L-Aminosäure, welches das Wirkenlassen einer von einer Pflanze abgeleiteten Phenylalanin-Ammoniak-Lyase auf ein Acrylsäurederivat der folgenden allgemeinen Formel (1).umfasst: 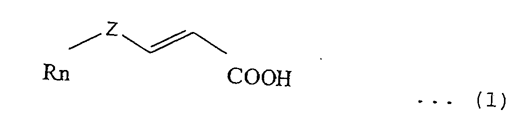
worin Z eine aromatische Ringgruppe darstellt, die ein Heteroatom aufweisen kann,
R einen Substituenten an dem aromatischen Ring darstellt und
n eine ganze Zahl von 0 oder höher ist und, wenn n 2 oder höher ist, die Substituenten R gleich oder unterschiedlich sein können;
wobei die L-Aminosäure durch die folgende allgemeine Formel (2) dargestellt ist: 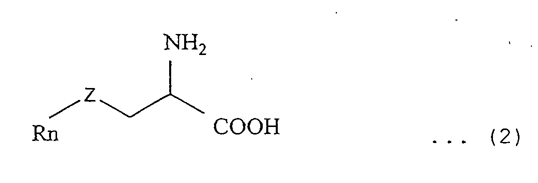 worin
Z eine aromatische Ringgruppe ist, die ein Heteroatom aufweisen kann,
R ein Substituent an dem aromatischen Ring ist,
n eine ganze Zahl von 0 oder höher ist und, wenn n 2 oder höher ist, die Substituenten R gleich oder unterschiedlich sein können.

2. Verfahren zur Herstellung einer L-Aminosäure nach Anspruch 1, worin Rn-Z- durch die folgende Formel (3) dargestellt ist: 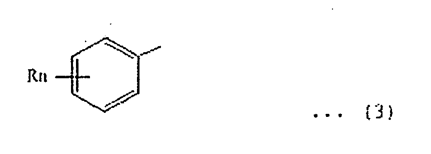 worin
R ein Substituent an einem Benzolring ist und eine Cyangruppe, Hydroxygruppe, Carboxygruppe, Amidgruppe, ein Fluoratom, Chloratom, Bromatom, eine Aminogruppe, Nitrogruppe, Hydroxymethylgruppe oder eine Alkyl- oder Alkoxygruppe mit 1 bis 6 Kohlenstoffatomen darstellt, und
n eine ganze Zahl von 0 bis 5 darstellt und, wenn n 2 oder höher ist, die Substituenten R gleich oder unterschiedlich sein können.

3. Verfahren zur Herstellung einer L-Aminosäure nach Anspruch 1, worin Rn-Z- durch die folgende allgemeine Formel (4) dargestellt ist: 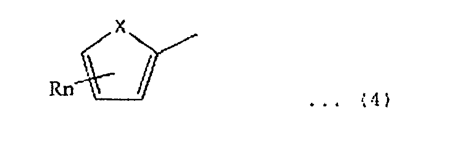 worin
X S, 0, NH oder NR¹ darstellt,
R¹ eine Alkylgruppe mit 1 bis 6 Kohlenstoffatomen darstellt,
R ein Substituent an einem Heteroring ist und eine Cyangruppe, Heteroxygruppe, Carboxygruppe, Amidgruppe, ein Fluoratom, Chloratom, Bromatom, eine Aminogruppe, Nitrogruppe, Hydroxymethylgruppe oder eine Alkyl- oder Alkoxygruppe mit 1 bis 6 Kohlenstoffatomen darstellt und
n eine ganze Zahl von 0 bis 3 ist und, wenn n 2 oder höher ist, die Substituenten R gleich oder unterschiedlich sein können.

4. Verfahren zur Herstellung einer L-Aminosäure nach Anspruch 1, worin Rn-Z- durch die folgende allgemeine Formel (5) dargestellt ist: 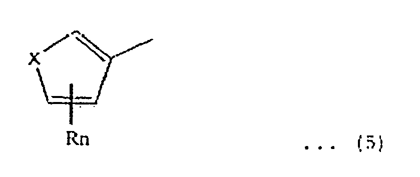 worin
X S, O, NH oder NR¹ ist,
R¹ eine Alkylgruppe mit 1 bis 6 Kohlenstoffatomen ist,
R ein Substituent an einem Heteroring ist und eine Cyangruppe, Hydroxygruppe, Carboxygruppe, Amidgruppe, ein Fluoratom, Chloratom, Bromatom, eine Aminogruppe, Nitrogruppe, Hydroxymethylgruppe oder eine Alkyl- oder Alkoxygruppe mit 1 bis 6 Kohlenstoffatomen darstellt und
n eine ganze Zahl von 0 bis 3 darstellt und, wenn n 2 oder höher ist, die Substituenten R gleich oder unterschiedlich sein können.

5. Verfahren zur Herstellung einer L-Aminosäure nach einem der Ansprüche 1 bis 4, welches den Einsatz einer Zusammensetzung, erhalten durch Behandeln von Pflanzengewebe, das eine Phenylalanin-Ammoniak-Lyase enthält, umfasst.

6. Verfahren zur Herstellung einer L-Aminosäure nach einem der Ansprüche 1 bis 4, welches den Einsatz einer kultivierten Pflanzenzelle, die eine Phenylalanin-Ammoniak-Lyase enthält, und/oder einer behandelten Zusammensetzung davon umfasst.

7. Verfahren zur Herstellung einer L-Aminosäure nach einem der Ansprüche 1 bis 4, welches das Zulassen der Exprimierbarkeit eines von einer Pflanze abgeleiteten Phenylalanin-Ammoniak-Lyasegens in einer Pflanze und den Einsatz von transformierten Pflanzenkulturen oder eines behandelten Produkts der Pflanzenkulturen umfasst.

8. Verfahren zur Herstellung einer L-Aminosäure nach einem der Ansprüche 1 bis 4, welches das Zulassen der Exprimierbarkeit eines von einer Pflanze abgeleiteten Phenylalanin-Ammoniak-Lyasegens in einem Mikroorganismus und den Einsatz einer transformierten Mikroorganismuskultur, eines behandelten Produkts davon oder eines von der Kultur erhaltenen Enzyms umfasst.

9. Verfahren zur Herstellung einer L-Aminosäure nach Anspruch 7 oder 8, wobei das von einer Pflanze abgeleitete Phenylalanin-Ammoniak-Lyasegen ein Gen ist, das für eine Aminosäuresequenz mit 70% oder höherer Homologie zu der Aminosäuresequenz kodiert, die von der Nukleotidsequenz des pa12-Gens aus Lithospermum erythrorhizon abgeleitet ist.

10. Verfahren zur Herstellung einer L-Aminosäure nach Anspruch 7 oder 8, wobei das von einer Pflanze abgeleitete Phenylalanin-Ammoniak-Lyasegen ein Gen ist, das für eine Aminosäuresequenz mit 80% oder höherer Homologie zu der Aminosäuresequenz kodiert, die von der Nukleotidsequenz des pa12-Gens aus Lithospermum erythrorhizon abgeleitet ist.

11. Verfahren zur Herstellung einer L-Aminosäure nach einem der Ansprüche 1 bis 8, wobei die Pflanze, von der das Phenylalanin-Ammoniak-Lyasegen abgeleitet ist, Lithospermum und/oder Camellia ist.

12. Verfahren zur Herstellung einer L-Aminosäure nach einem der Ansprüche 8 bis 11, wobei der Mikroorganismus ein Bakterium, Hefe und/oder ein filamentöser Pilz ist.

13. Verfahren zur Herstellung einer L-Aminosäure nach Anspruch 12, wobei das Bakterium Escherichia coli ist.

14. Verfahren zur Herstellung einer L-Aminosäure nach einem der Ansprüche 2 und 5 bis 13, wobei in der allgemeinen Formel (3) mindestens einer der Substituenten R eine Hydroxygruppe ist.

15. Verfahren zur Herstellung einer L-Aminosäure nach einem der Ansprüche 2 und 5 bis 13, wobei in der allgemeinen Formel (3) mindestens einer der Substituenten R eine Cyangruppe ist.

16. Verfahren zur Herstellung einer L-Aminosäure nach einem der Ansprüche 2 und 5 bis 13, wobei in der allgemeinen Formel (3) mindestens einer der Substituenten R eine Carboxygruppe ist.

17. Verfahren zur Herstellung einer L-Aminosäure nach einem der Ansprüche 2 und 5 bis 13, wobei in der allgemeinen Formel (3) mindestens einer der Substituenten R eine Amidgruppe ist.

18. Verfahren zur Herstellung einer L-Aminosäure nach einem der Ansprüche 2 und 5 bis 13, wobei in der allgemeinen Formel (3) mindestens einer der Substituenten R eine Halogengruppe ist.

19. Verfahren zur Herstellung einer L-Aminosäure nach einem der Ansprüche 2 und 5 bis 13, wobei in der allgemeinen Formel (3) mindestens einer der Substituenten R eine Aminogruppe ist.

20. Verfahren zur Herstellung einer L-Aminosäure nach einem der Ansprüche 2 und 5 bis 13, wobei in der allgemeinen Formel (3) mindestens einer der Substituenten R eine Nitrogruppe ist.

21. Verfahren zur Herstellung einer L-Aminosäure nach einem der Ansprüche 2 und 5 bis 13, wobei in der allgemeinen Formel (3) mindestens einer der Substituenten R eine Hydroxymethylgruppe ist.

22. Verfahren zur Herstellung einer L-Aminosäure nach einem der Ansprüche 2 und 5 bis 13, wobei in der allgemeinen Formel (3) mindestens einer der Substituenten R eine Alkylgruppe mit 1 bis 6 Kohlenstoffatomen ist.

23. Verfahren zur Herstellung einer L-Aminosäure nach einem der Ansprüche 1 bis 13, wobei R eine unter einer Cyangruppe, Hydroxygruppe, Nitrogruppe und Carboxygruppe ausgewählte Gruppe ist und n 1 ist.

24. Verfahren zur Herstellung einer L-Aminosäure nach einem der Ansprüche 2 und 5 bis 13, wobei die L-Aminosäure L-Phenylalanin ist.

25. Verfahren zur Herstellung einer L-Aminosäure nach einem der Ansprüche 3 und 5 bis 13, wobei X in der allgemeinen Formel (4) 0 ist.

26. Verfahren zur Herstellung einer L-Aminosäure nach einem der Ansprüche 3 und 5 bis 13, wobei X in der allgemeinen Formel (4) S ist.

27. Verfahren zur Herstellung einer L-Aminosäure nach einem der Ansprüche 3 und 5 bis 13, wobei X in der allgemeinen Formel (4) NH ist.

28. Verfahren zur Herstellung einer L-Aminosäure nach einem der Ansprüche 4 bis 13, wobei X in der allgemeinen Formel (5) O ist.

29. Verfahren zur Herstellung einer L-Aminosäure nach einem der Ansprüche 4 bis 13, wobei X in der allgemeinen Formel (5) S ist.

30. Verfahren zur Herstellung einer L-Aminosäure nach einem der Ansprüche 4 bis 13, wobei X in der allgemeinen Formel (5) NH ist.

## Revendications

1. Procédé de production d'un L-aminoacide, qui comprend, en présence d'ammoniac, de permettre à une phénylalanine ammonia-lyase dérivée de plante d'agir sur un dérivé d'acide acrylique représenté par la formule générale suivante (1) : 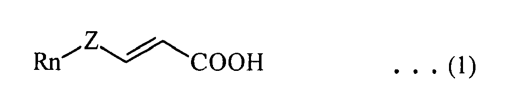 dans laquelle
Z représente un groupe cyclique aromatique qui peut comprendre un hétéroatome,
R représente un substituant dudit cycle aromatique, et n représente un nombre entier égal ou supérieur à 0, et où n est 2 ou supérieur, chaque R peut être identique ou différent ;
ledit L-aminoacide étant représenté par la formule générale suivante (2) : 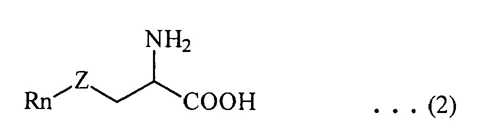 dans laquelle
Z représente un groupe cyclique aromatique qui peut comprendre un hétéroatome,
R représente un substituant dudit cycle aromatique, et n représente un nombre entier égal ou supérieur à 0, et où n est 2 ou supérieur, chaque R peut être identique ou différent.

2. Procédé de production d'un L-aminoacide selon la revendication 1, **caractérisé en ce que** Rn-Z est représenté par la formule générale suivante (3) : 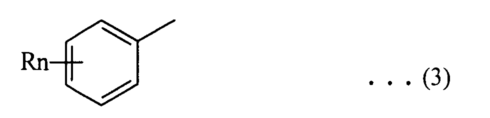 dans laquelle
R représente un substituant sur un cycle benzénique et représente un groupe cyano, un groupe hydroxyle, un groupe carboxyle, un groupe amide, un atome de fluor, un atome de chlore, un atome de brome, un groupe amino, un groupe nitro, un groupe hydroxyméthyle, ou un groupe alkyle ou alcoxy ayant 1 à 6 atomes de carbone, et
n représente un nombre entier de 0 à 5 et où n est 2 ou supérieur, chaque R étant identique ou différent.

3. Procédé de production d'un L-aminoacide selon la revendication 1, **caractérisé en ce que** Rn-Z est représenté par la formule générale suivante (4) : 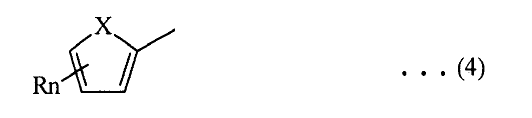 dans laquelle
X représente S, O, NH ou NR¹,
R¹ représente un groupe alkyle ayant 1 à 6 atomes de carbone,
R est un substituant sur un hétérocycle et représente un groupe cyano, un groupe hydroxyle, un groupe carboxyle, un groupe amide, un atome de fluor, un atome de chlore, un atome de brome, un groupe amino, un groupe nitro, un groupe hydroxyméthyle, ou un groupe alkyle ou alcoxy ayant 1 à 6 atomes de carbone, et
n représente un nombre entier de 0 à 3 et où n est 2 ou supérieur, chaque R étant identique ou différent.

4. Procédé de production d'un L-aminoacide selon la revendication 1, **caractérisé en ce que** Rn-Z est représenté par la formule générale suivante (5) : 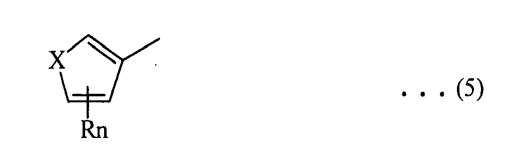 dans laquelle
X représente S, O, NH ou NR¹,
R¹ représente un groupe alkyle ayant 1 à 6 atomes de carbone,
R est un substituant sur un hétérocycle et représente un groupe cyano, un groupe hydroxyle, un groupe carboxyle, un groupe amide, un atome de fluor, un atome de chlore, un atome de brome, un groupe amino, un groupe nitro, un groupe hydroxyméthyle, ou un groupe alkyle ou alcoxy ayant 1 à 6 atomes de carbone, et
n représente un nombre entier de 0 à 3 et où n est 2 ou supérieur, chaque R étant identique ou différent.

5. Procédé de production d'un L-aminoacide selon l'une quelconque des revendications 1 à 4, qui comprend l'utilisation d'une composition obtenue par le traitement de tissus de plante contenant une phénylalanine ammonia-lyase.

6. Procédé de production d'un L-aminoacide selon l'une quelconque des revendications 1 à 4, qui comprend l'utilisation d'une cellule de plante cultivée contenant une phénylalanine ammonia-lyase et/ou une composition traitée de celle-ci.

7. Procédé de production d'un L-aminoacide selon l'une quelconque des revendications 1 à 4, qui comprend la possibilité de permettre à un gène de phénylalanine ammonia-lyase dérivé d'une plante d'être exprimable dans une plante et utilisation des cultures de plante transformée ou un produit traité des cultures de plante.

8. Procédé de production d'un L-aminoacide selon l'une quelconque des revendications 1 à 4, qui comprend la possibilité de permettre à un gène de phénylalanine ammonia-lyase dérivé d'une plante d'être exprimable dans un micro-organisme et utilisation de la culture de micro-organisme transformé, un produit traité de celle-ci ou un enzyme obtenu à partir de la culture.

9. Procédé de production d'un L-aminoacide selon l'une quelconque des revendications 7 à 8, **caractérisé en ce que** le gène de phénylalanine ammonia-lyase dérivé d'une plante est un gène qui code une séquence d'aminoacide qui a 70 % ou plus d'homologie avec la séquence d'aminoacide déduite de la séquence de nucléotides du gène pa12 dérivé de *Lithospermum erythrorhizon.*

10. Procédé de production d'un L-aminoacide selon l'une quelconque des revendications 7 à 8, **caractérisé en ce que** le gène de phénylalanine ammonia-lyase dérivé d'une plante est un gène qui code une séquence d'aminoacide qui a 80 % ou plus d'homologie avec la séquence d'aminoacide déduite de la séquence de nucléotides du gène pa12 dérivé de *Lithospermum erythrorhizon.*

11. Procédé de production d'un L-aminoacide selon l'une quelconque des revendications 1 à 8, **caractérisé en ce que** la plante dont est dérivé le gène de phénylalanine ammonia-lyase est *Lithospermum* et/ou *Camellia.*

12. Procédé de production d'un L-aminoacide selon l'une quelconque des revendications 8 à 11, **caractérisé en ce que** le micro-organisme est une bactérie, une levure et ou un mycète filamenteux.

13. Procédé de production d'un L-aminoacide selon la revendications 12, **caractérisé en ce que** la bactérie est *Eschrichia coli.*

14. Procédé de production d'un L-aminoacide selon l'une quelconque des revendications 2 et 5 à 13, **caractérisé en ce que**, dans ladite formule générale (3), au moins un des substituants R est un groupe hydroxyle.

15. Procédé de production d'un L-aminoacide selon l'une quelconque des revendications 2 et 5 à 13, **caractérisé en ce que**, dans ladite formule générale (3), au moins un des substituants R est un groupe cyano.

16. Procédé de production d'un L-aminoacide selon l'une quelconque des revendications 2 et 5 à 13, **caractérisé en ce que**, dans ladite formule générale (3), au moins un des substituants R est un groupe carboxyle.

17. Procédé de production d'un L-aminoacide selon l'une quelconque des revendications 2 et 5 à 13, **caractérisé en ce que**, dans ladite formule générale (3), au moins un des substituants R est un groupe amide.

18. Procédé de production d'un L-aminoacide selon l'une quelconque des revendications 2 et 5 à 13, **caractérisé en ce que**, dans ladite formule générale (3), au moins un des substituants R est un atome d'halogène.

19. Procédé de production d'un L-aminoacide selon l'une quelconque des revendications 2 et 5 à 13, **caractérisé en ce que**, dans ladite formule générale (3), au moins un des substituants R est un groupe amino.

20. Procédé de production d'un L-aminoacide selon l'une quelconque des revendications 2 et 5 à 13, **caractérisé en ce que**, dans ladite formule générale (3), au moins un des substituants R est un groupe nitro.

21. Procédé de production d'un L-aminoacide selon l'une quelconque des revendications 2 et 5 à 13, **caractérisé en ce que**, dans ladite formule générale (3), au moins un des substituants R est un groupe hydroxyméthyle.

22. Procédé de production d'un L-aminoacide selon l'une quelconque des revendications 2 et 5 à 13, **caractérisé en ce que**, dans ladite formule générale (3), au moins un des substituants R est un groupe alkyle ayant 1 à 6 atomes de carbone.

23. Procédé de production d'un L-aminoacide selon l'une quelconque des revendications 1 à 13, **caractérisé en ce que** R est n'importe quel groupe cyano, groupe hydroxyle, groupe nitro et groupe carboxyle et n est 1.

24. Procédé de production d'un L-aminoacide selon l'une quelconque des revendications 2 et 5 à 13, **caractérisé en ce que** le L-aminoacide est la L-phénylalanine.

25. Procédé de production d'un L-aminoacide selon l'une quelconque des revendications 3 et 5 à 13, **caractérisé en ce que** dans la formule générale (4), X est O.

26. Procédé de production d'un L-aminoacide selon l'une quelconque des revendications 3 et 5 à 13, **caractérisé en ce que** dans la formule générale (4), X est S.

27. Procédé de production d'un L-aminoacide selon l'une quelconque des revendications 3 et 5 à 13, **caractérisé en ce que** dans la formule générale (4), X est NH.

28. Procédé de production d'un L-aminoacide selon l'une quelconque des revendications 4 à 13, **caractérisé en ce que** dans la formule générale (5), X est O.

29. Procédé de production d'un L-aminoacide selon l'une quelconque des revendications 4 à 13, **caractérisé en ce que** dans la formule générale (5), X est S.

30. Procédé de production d'un L-aminoacide selon l'une quelconque des revendications 4 à 13, **caractérisé en ce que** dans la formule générale (5), X est NH.


### SEQUENCE LISTING

<110> SHOWA DENKO K.K.
   <120> Biologycal Production of Phenylalanine Derivatives
   <130> Primers for PAL
<140>
   <141>
   <160> 6
   <170> PatentIn Ver. 2.1
   <210> 1
   <211> 23
   <212> DNA
   <213> Lithospermum erythrorhizon
   <400> 1
   aagatatcat ggaaaccata gtg 23
   <210> 2
   <211> 23
   <212> DNA
   <213> Lithospermum erythrorhizon
   <400> 2
   ttgtcgactt aacagattgg aag 23
   <210> 3
   <211> 25
   <212> DNA
   <213> Lithospermum erythrorhizon
   <400> 3
   aagaattcat ggaaaatgga aatgg 25
   <210> 4
   <211> 23
   <212> DNA
   <213> Lithospermum erythrorhizon
   <400> 4
   ttgtcgacta acatattgga aga 23
   <210> 5
   <211> 23
   <212> DNA
   <213> Camellia sinensis
   <400> 5
   aacccgggat ggatagtacc ace 23
   <210> 6
   <211> 23
   <212> DNA
   <213> Camellia sinensis
   <400> 6
   ttaagcttct aacagatagg aag 23